# EUROPEAN PATENT SPECIFICATION

(11) **EP 3 959 729 B1**
(45) Date of publication and mention of the grant of the patent: **04.03.2026**
(21) Application number: 20795661.6
(22) Date of filing: 02.04.2020
(51) Int. Cl.: G16H 80/00, G16H 40/00, G16H 15/00, G16H 40/63, G06F 3/048, H04M 1/2747, H04M 1/57, H04M 3/42, H04M 1/72469

(54) **PROVIDING CONTEXT INFORMATION WITH A COMMUNICATION REQUEST**
BEREITSTELLUNG VON KONTEXTINFORMATIONEN MIT EINER KOMMUNIKATIONSANFORDERUNG
FOURNITURE D'INFORMATIONS DE CONTEXTE AVEC DEMANDE DE COMMUNICATION

(30) Priority: 22.04.2019 US 201916390351
(43) Date of publication of application: 02.03.2022
(73) Proprietor: Stryker Corporation, Portage, MI 49002-9711 (US)
(72) Inventor: HOLSCHER, David, Portage, Michigan 49002-9711 (US); BAYS, Justin, Portage, Michigan 49002-9711 (US); MIRCHANDANI, Arun, Portage, Michigan 49002-9711 (US)
(74) Representative: Graham Watt & Co
(86) International application number: PCT/US2020/026315
(87) International publication number: WO 2020/219250

(56) References cited:
- US-A1- 2005 146 431
- US-A1- 2008 091 473
- US-A1- 2013 183 923
- US-A1- 2017 032 093
- US-A1- 2017 308 650
- US-A1- 2017 352 238
- US-A1- 2019 108 908
- US-A1- 2019 110 763

## Description

### BACKGROUND

Wireless communication systems are increasingly utilized to facilitate coordination among workgroups in a variety of environments. Wireless communication systems have proven highly efficacious in hospitals and other healthcare environments, because mobile communicators enable rapid communication among doctors, nurses, and other care team staff who sometimes must provide time-critical patient care. Healthcare environments can be busy, information rich, stressful and distracting. Care providers receive a torrent of information and requests for their attention. Pages, voice calls and public announcements demanding care giver attention may range from the mundane to time-critical emergencies involving the life or health of patients. In such environments, a recipient of an incoming call or page who is otherwise engaged in a healthcare task must decide whether to interrupt the task to answer the call/page. Without more information than the name or phone number of the caller (e.g., caller ID), recipients may ignore a call or page that is urgen

US 2005/146431 describes patient monitoring systems commonly used in hospitals and clinics, which collect and report patient health data (such as vital signs and live sensor data) and provide context information to caregivers through wireless devices, enabling functions like notifications, voice calls, patient identification, and automatic updates on staffing changes. The system allows data forwarding: a caregiver receiver can transmit or request the forwarding of displayed alert data to a second caregiver device, either directly or via a server, and alert data may be sent alongside a voice call or through a single user action.

### SUMMARY

Various embodiments provide methods, systems, wireless communication devices, and non-transitory process-readable storage media for providing recipients of an incoming communication with information regarding the context and/or purposes of the call ("call context information"). Various embodiments may include receiving, by a processor of a server device, clinical information in one or more information feeds from one or more other devices, determining, by the server device, whether a communication request from a first communication device for a second communication device has been received, and sending, by the server device, a communication request message including call context information to the second communication device, wherein the call context information is based on the clinical information.

Some embodiments may further include determining, by the server device, an event identifier associated with the communication request, and determining, by the server device, the call context information based on the event identifier associated with the communication request.

Some embodiments may further include associating, by the server device, clinically relevant elements of the clinical information with one or more event identifiers. Some embodiments may further include formatting, by the processor, such clinical information into a format for transmission as part of the call context information provided to the second communication device. Some embodiments may further include storing, by the processor, the association of the elements of clinical information and the one or more event identifiers in a database.

In some embodiments, sending a communication request message including call context information to the second communication device may include obtaining, by the server device, elements of clinical information associated with an event identifier associated with the communication request received from the first communication device. In such embodiments, the call context information included in the communication request message sent to the second communication device may include one or more of the elements of the clinical association associated with the event identifier.

Some embodiments may further include determining whether a patient event alert has been received or should be issued based upon the received clinical information, and sending, by the server device, to the first communication device an event alert in response to determining that a patient event alert has been received or should be issued, the event alert including an event identifier and context information.

Some embodiments may further include: receiving, by the first communication device, the event alert; displaying, by the first communication device, a graphical user interface (GUI) display identifying the event and the context information along with user interface icons for accepting or declining the event alert; displaying, by the first communication device, a GUI display to enable a caregiver to place a communication request related to the event alert; and transmitting, by the first communication device, to the server device a communication request related to the event alert in response to receiving a user input to place the communication request.

Some embodiments may further include receiving, by the second communication device, the communication request message and call context information, displaying, by the second communication device, a GUI display identifying the event and the context information along with user interface icons for accepting or declining the communication request, and transmitting, by the second communication device, to the server device a message to accept the communication request in response to receiving a user input to accept the communication request.

Further embodiments include a server device having a processor configured with processor-executable instructions to perform operations of any of the methods summarized above. Further embodiments include a communication system including a server device, a first communication device, and a second communication device, each including a processor configured with processor-executable instructions to perform operations of any of the methods summarized above. Further embodiments include a non-transitory processor-readable medium on which is stored processor-executable instructions configured to cause a processor of a server device to perform operations of any of the methods summarized above. Further embodiments include a communication system including a communication device having means for performing functions of any of the methods summarized above.

### BRIEF DESCRIPTION OF THE DRAWINGS

The accompanying drawings, which are incorporated herein and constitute part of this specification, illustrate exemplary embodiments of the invention, and together with the general description given above and the detailed description given below, serve to explain the features of the invention.
FIG. 1 is a component block diagram of a communication system suitable for use in various embodiments.
FIGS. 2-9 illustrate operations of a communication device suitable for use in some embodiments.
FIG. 10 is a process flow diagram illustrating a method of providing call context information according to some embodiments.
FIG. 11 is a data flow diagram illustrating a method of providing call context information according to some embodiments.
FIGS. 12-17 illustrate operations of a communication device suitable for use in some embodiments.
FIG. 18 is a process flow diagram illustrating a method of providing call context information according to some embodiments.
FIG. 19 is a data flow diagram illustrating a method of providing call context information according to some embodiments.
FIG. 20 is a component block diagram of a communication device suitable for use in some embodiments.
FIG. 21 is a component block diagram of a server computing device suitable for use in some embodiments.

### DETAILED DESCRIPTION

The various embodiments will be described in detail with reference to the accompanying drawings. Wherever possible, the same reference numbers will be used throughout the drawings to refer to the same or like parts. References made to particular examples and implementations are for illustrative purposes, and are not intended to limit the scope of the invention or the claims.

Various embodiments provide methods and communication devices and systems for providing call context information to a communication device as part of a communication request in a healthcare environment. As used herein, the term "call context information" refers to information displayed on a wireless communication device as part of an incoming call announcement (e.g., a display presented when the device is ringing) that informs the user of a medical or patient condition, medical event, purpose or other context of the call. Various embodiments include methods and systems for automatically assembling the call context information from a variety of sources (e.g., information feeds from various server devices, clinical information sources such as sensor devices, patient monitoring systems, and the like) relevant to a reason that the call is being placed, as well as formatting the information in a manner useful to the recipient of an incoming call. In some embodiments, the call context information may be associated with an identifier of a medical event (referred to herein as an "event identifier") that is a reason for or otherwise relevant to the call being placed. In some embodiments, a communication request sent to a communication device may include or be sent with an event identifier and associated call context information. In various embodiments, the receiving communication device may display (e.g., via an output device) the call context information and a notification of the incoming communication request. The presentation of the call context information and the communication request notification may enable a call recipient to evaluate the nature and/or urgency of the communication request, and thus determine whether to answer the call.

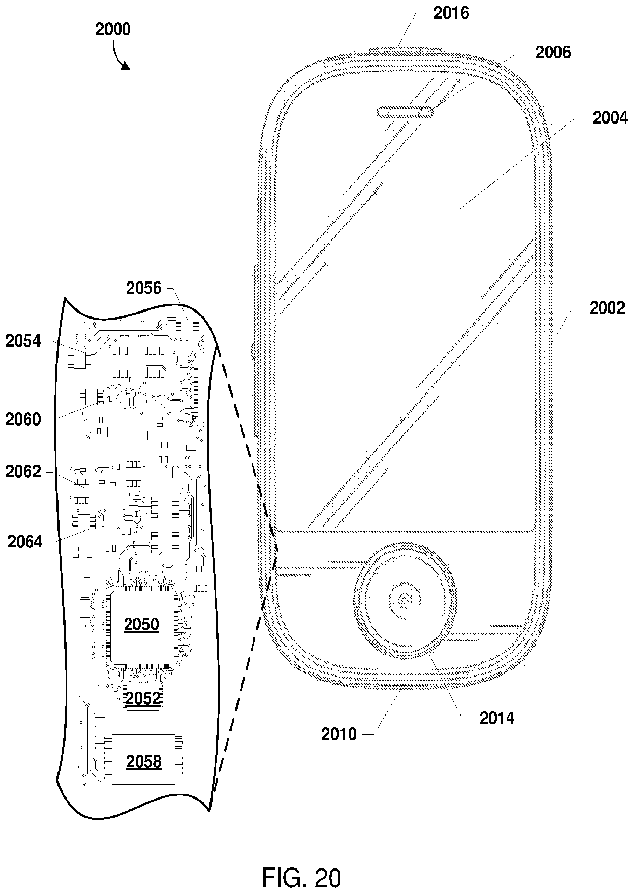

The term "communication device" is used herein to refer to an electronic device equipped with at least a processor and a transceiver configured to support wireless communications with a wireless local area network (WLAN). Examples of communication devices include mobile devices, particularly communicators for use within a hospital or other healthcare environments. In various embodiments, communication devices may be configured with memory or storage as well as networking capabilities, such as network transceiver(s) and antenna(s) configured to establish a WLAN connection with an access point. Communication devices may also include voice communications badge devices, an example of which is illustrated in FIG. 20.

As noted above, healthcare providers work in busy environments and may be a bombarded with information and requests for their attention ranging from mundane communications to urgent communications involving the life or health of a patient. Thus, care providers may ignore a page or phone call when engaged in an important or urgent medical task because a page or call provides no information regarding whether the message or call is urgent or mundane. For example, busy caregivers may ignore an incoming call, wait until a message is left, then listen to the message to determine whether the matter is important enough to interrupt current activities. By doing this, the caregiver may lose precious time for responding to an urgent medical situation.

Various embodiments enable the assembly and provisioning of call context information to a communication device receiving a communication request. The call context information may be provided to a user together with the communication request to enable the user to evaluate aspects of the communication request, such as the reason for the communication request and/or the urgency of the communication request.

Various embodiments automated methods of gathering clinical information relevant to an outgoing call for use in assembling the call context information to be sent along with a communication request to a recipient's mobile device. For example, automated methods may gather patent and clinical information relevant to the context of a call from electronic medical records (EMR), associations of health care providers and patients (e.g., care team assignments), information from patient or environmental sensors (e.g., nurse call systems, patient physiological monitors, video surveillance systems, bed exit monitors, vital sign monitoring devices), identities of care providers in the vicinity of the patient or caller, and other information. Such information is referred to generally herein as "clinical information." As used herein, clinical information is not limited to patient-specific information, and may include environmental information (e.g., room temperature, humidity, and other suitable information), non-medical staffing information (e.g., personnel with maintenance, janitorial, security, information technology, or other roles), reports made about conditions in a facility (e.g., a report that a waiting room is uncomfortably cold), and other information that may be useful to a recipient of an incoming call or page. In various embodiments, the type, number, content, etc. of clinical information may be configured for any type of use or application as desired.

In some embodiments, a server device may receive clinical information from one or more other network elements in a system. In some embodiments, the server device may be configured to receive the clinical information in one or more information feeds. Information feeds may be provided by or obtained from, for example, an EMR system, a staffing or personnel system, one or more sensors/sources (e.g., a patient physiological monitor, an environmental sensor, a nurse call button, a bed exit monitor, or other suitable information source), an event reporting system, or another suitable network element. By receiving information feeds from various network elements, the server device may avoid interfering with or otherwise altering the normal functions and efficiency of the other network elements. For example, the server device may not directly access electronic medical records stored on an EMR system, but rather may receive such information provided by the EMR system to the server device to enable a duplicate copy of the information to be used for determining call context information.

The server device may provide the call context information to a communication device to supplement a communication request (e.g., ring and caller ID) sent to the communication device. For example, a first communication device of a care team member may receive an event (e.g., in the form of an alert about a patient condition or occurrence). The event is associated with an event identifier. The event may be associated with context information that is also sent to the first communication device. In some embodiments, the event may be associated with a number of potential recipients of a communication request that the first communication device may send (e.g., other members of a patient's care team). The care team member may provide an input (e.g., press a button or touch a user interface icon) to the first communication device to call (i.e., send a communication request to) a second care team member related to the event, and in response the first communication device may send the call request and the event identifier to a server device. The server device may obtain the call context information for or related to the event identifier, and send to the second care team member's communication device (a second communication device) the communication request along with the call context information. The communication request and the call context information may be sent in one or more messages, together or separately, as part of the process for calling the second communication device. The server device may determine and send the call context information automatically, so that the user of the first communication device (i.e., the caller) does not need to determine or choose the information to provide or include in the call context information.

In various embodiments, and event identifier may identify a patient event (e.g., a patient condition, a patient change, a patient occurrence, or another suitable condition), an environmental event (e.g., a room temperature meeting a threshold, a security alert, or another suitable condition), or another suitable event or occurrence. In some embodiments, the server device may associate with an event identifier one or more elements of the clinical information that are clinically relevant to the identified event. For example, a given patient event, such as an alert that a patient has low blood oxygen saturation levels, may be associated with a variety of clinical information sources, such as other patient monitors (e.g., pulse monitor or EKG, temperature, blood pressure, etc.). Associated clinical information may also include patient information, such as the patient's room number, name, age, sex, admittance condition, latest measurements of vital signs, time that the vital signs were measured, and the like. The associated information elements may also include prior information, such as medical history, event histories, a log of, or access to a log of, notes, text communications, and other information that may be related to the given event. In various embodiments, the server device may determine different context information to be sent with a communication request depending on recipient of the communication request and/or the event.

The second communication device may receive the communication request and the call context information, and present the call context information on a display while providing the income call alert (e.g., ringing, vibrating and/or flashing). For example, the second communication device may display the call context information while sounding a ring tone. The second communication device may receive a user input (e.g., a button press or touch on a user interface icon) to accept the communication request, and exchange wireless messages with the server device to accept the call and establish a voice communication session via the network.

Various embodiments improve the operation of a communication system by automatically providing call context information to a communication device along with a communication request, thereby informing the call recipient about clinically relevant information regarding the incoming call. Providing such call context information may increase the efficacy and usefulness of the communication system, especially in stressful, information rich environments. Various embodiments facilitate the communication of relevant information to a call recipient before a voice communication session is established. Various embodiments reduce the burden callers by automatically compiling and sending call context information relevant to the communication request to the receiving communication device. Further, various embodiments improve communication systems by reducing the time required to convey relevant and/or time-sensitive information from one communication device to another.

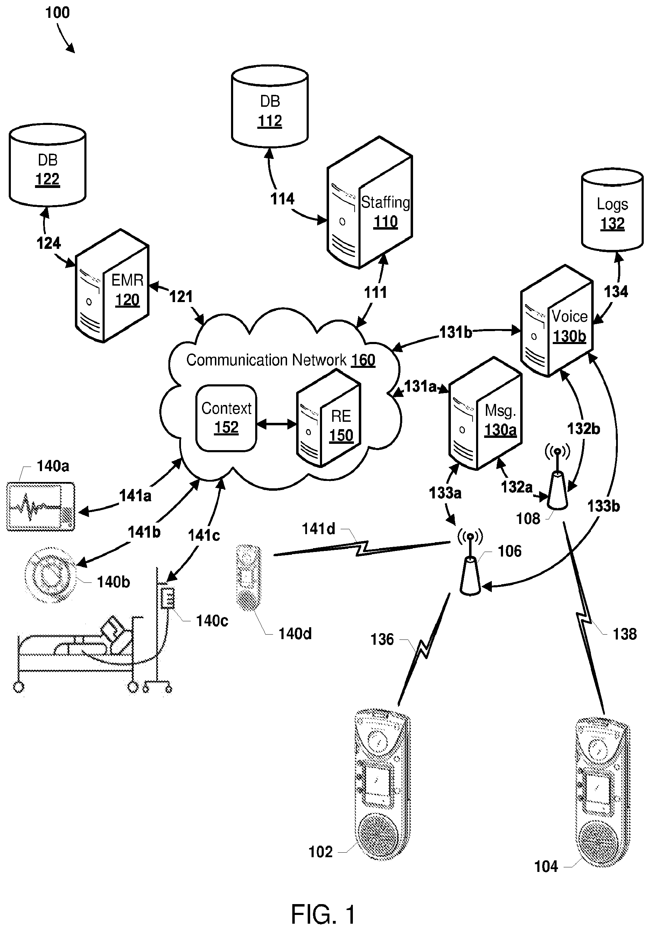

FIG. 1 illustrates a communication system 100 suitable for use with the various embodiments. The communication system 100 may include communication devices 102, 104, a staffing server 110, an electronic medical record (EMR) server 120, a messaging server 130a, a voice communications server 130b, one or more sensors/sources 140a-140d, and a rules engine 150. The various elements of the communication system 100 may be configured to communicate over a communication network 160 via wired or wireless communication links 111, 121, 131a, 131b, and 141a-141d. In some embodiments, one or more of the staffing server 110, the EMR server 120, the messaging server 130a, the voice communications server 130b, and the rules engine 150 may be configured as separate devices (e.g., server devices). In some embodiments, one or more of the staffing server 110, the EMR server 120, the messaging server 130a, the voice communications server 130b, and the rules engine 150 may be configured as separate logical services on one server or similar device.

The EMR server 120 may include one or more server computing devices configured to store, update, and transmit information such as patient-based data. The EMR server 120 may communicate over a wired or wireless communication link 124 with a database 122 configured to store data records. Patient-based data may include identifiers or codes indicating an identity of a patient, health care personnel associated with the patient (e.g., physician, specialist, hospitalist, nurse, etc.), patient location information (e.g., room, bed, wing, building), a status of the patient (e.g., discharged, admitted, etc.), and other suitable information. The EMR server 120 may transmit messages (e.g., in HL7 or another suitable format) including patient-based data via one or more information feeds. In some embodiments, the EMR server 120 may transmit the messages on the occurrence of an event that changes the patient-based data at the EMR server 120. For example, the EMR server 120 may transmit a message that indicates a patient identifier and a room identifier in response to the patient corresponding to the patient identifier being admitted to the hospital and being assigned to a room corresponding to the room identifier. In some embodiments, the EMR server 120 may be connected to or otherwise may utilize a system capable of sending and receiving HL7 version 2.3 messages (e.g., ADT messages), such as messages that include a role (or "ROL") segment that indicates care team assignment information. The EMR server 120 may transmit information (e.g., in HL7, ADT messaging, or another suitable format) via one or more information feeds (e.g., to the rules engine 150).

The staffing server 110 may be one or more server computing devices configured to at least synchronize care team assignment data from different systems related to the hospital. The staffing server 110 may communicate over a wired or wireless communication link 114 with a database 112 configured to store data records. In some embodiments, the staffing server 110 may be configured to continually receive data from the EMR server 120, the voice communications server 130, and/or other systems that indicate staffing changes (e.g., to care teams associated with the various patients, locations, and/or shifts of the hospital). For example, the staffing server 110 may receive subscription messages from the voice communications server 130 indicating when particular nurses of the hospital log-in or out of a shift and/or HL7 messages from the EMR server 120 that indicate when a particular patient's data changes (e.g., assigned to a new bed, room, specialist doctor, etc.). The data records may include, e.g., records related to the various patients admitted to a hospital and/or the various care teams active in the hospital, and may be accessed to obtain a data record indicating the last known nurse, nurse assistant, bed, wing, building, physician, specialist, and hospitalist for a particular patient identifier. The staffing server 110 may transmit information (e.g., in HL7, ADT messaging, or another suitable format) via one or more information feeds (e.g., to the rules engine 150).

The messaging server 130a may include one or more server computing devices configured to control various messages sent between the communication devices 102, 104 via access points 106, 108. In some embodiments, the messaging server 130a may be configured to control messages sent to the communication devices 102, 104 from the rules engine 150.

The voice communications server 130b may include one or more server computing devices configured to control various voice calls placed between the communication devices 102, 104 via access points 106, 108. In some embodiments, the voice communications server 130b may include a signaling gateway service to facilitate communications between and among the communication devices 102, 104 and the voice communications server 130b, such as login functions, voice call functions, and other suitable functions. In some embodiments, the signaling gateway service may be configured as a separate device (not illustrated). As noted above, in various embodiments, the messaging server 130a and the voice communications server 130b may be configured as separate devices, or as logical functions in one device. In some embodiments, the messaging server 130a and the voice communications server may transmit information in a suitable format via one or more information feeds (e.g., to the rules engine 150).

In operation, the communication system 100 may include a large number of communication devices and access points, illustrated as communication devices 102, 104, 140d and access points 106, 108 for conciseness. The communication devices 102, 104, 140d may communicate with an access point 106, 108 over separate wireless communication links 120, 122, 124. The access points 106, 108 may communicate with the voice communications server 130 over separate communication links 128, 130, 141d. The voice communications server 130 may control various messages and voice calls placed between the communication devices 102, 104, 140d. The voice communications server 130 may communicate over a wired or wireless communication link 134 with a database 132 configured to store logs and other data records.

In some embodiments, the voice communications server 130b may be configured to provide information to the rules engine 150via one or more information feeds. For example, the voice communications server 130 may store, update, and transmit at least shift-based and/or location-based data of the various care team assignments of the hospital. The voice communications server 130 may also store, update, and transmit patient-related information, information related to the facility or environment, and other information. For example, the voice communications server 130 may receive messages from any of the communication devices 102, 104 that indicate users of the communication devices 102, 104, 141d have logged-out of or logged-into a shift of working in a care team at the hospital. As another example, the voice communications server 130 may receive a message from a communication device 102, 104, 141d regarding the condition of a patient, equipment, a location, an environmental condition, or other suitable information. The voice communications server 130 may transmit information in a suitable format via one or more information feeds (e.g., to the rules engine 150).

The one or more sensors/sources 140a-140d may include one or more sensor devices to sense information about a patient, an environment, or other suitable information. The one or more sensors/sources 140a-140d may further include one or more sources information about a patient, an environment, or other suitable information, such as a bed exit monitor, a nurse call button/system, a video surveillance system, or another suitable source). For example, patient monitors 140a, 140c may include devices configured to monitor one or more patient conditions or vital signs. Room sensors 140b may be configured to sense and provide information about one or more environmental conditions, or aspects of a person or object to which the sensor is attached (e.g., temperature, humidity, motion, door or window security, ambient light conditions, location, acceleration, orientation, etc.) Communication devices 140d may also function as a source of clinical or call context information, such as identifying users of the devices (i.e., caregivers) in proximity to a patient. In some embodiments, the one or more sensors/sources 140a-140d may be configured with, or may communication with a device configured with, a processor and a wired or wireless communication capability to communicate sensed information in a suitable format over a wired or wireless communication links 141a-141d. In some embodiments, the one or more sensors/sources 140a-140d may transmit information in a suitable format via one or more information feeds to the rules engine 150.

The rules engine 150 may include one or more server computing devices configured to receive clinical information via various information feeds from other network elements such as the staffing server 110, the EMR server 120, and the one or more sensors/sources 140a-140d. In various embodiments, the various network elements (e.g., the staffing server 110, the EMR server 120, and the one or more sensors/sources 140a-140d) may be configured to send information to the rules engine 150 in an unsolicited manner (e.g., without requiring a query or another message soliciting information). By receiving information feeds from the other network elements, the rules engine 150 may avoid interfering with or otherwise altering the normal function and efficiency of the other network elements. For example, the rules engine 150 may not alter electronic medical records stored on the EMR server 120, but rather may receive information periodically provided by the EMR server 120 and stored on the rules engine 150. The rules engine 150 may be configured to associate certain portions or element(s) of the clinical information with one or more event identifiers for use in generating call context information for an event identifier associated with a particular communication request (i.e., call). Further, the rules engine 150 may be configured to provide the call context information to a called communication device 102, 104, e.g., when a communication request is sent to a communication device 102, 104, as further described below.

The communication links 111, 114, 121, 124, 131a-134, and 141a-141d may include wired or wireless communication links. Wired communication links may include, for example, twisted pair cable, coaxial cable or fiber optic cable, or combinations thereof. Wireless communication links may include a radio frequency, microwave, infrared, or other similar signal. Wireless communication links may include a plurality of carrier signals, frequencies, or frequency bands, each of which may include a plurality of logical channels. For example, wireless communication links may be established over a Wi-Fi local area wireless communication network.

Other network elements may be present in a communication system 100 system to facilitate communications are omitted for clarity, including additional access points, processing nodes, routers, gateways, and other network elements, as well as physical and/or wireless data links for carrying signals among the various network elements.

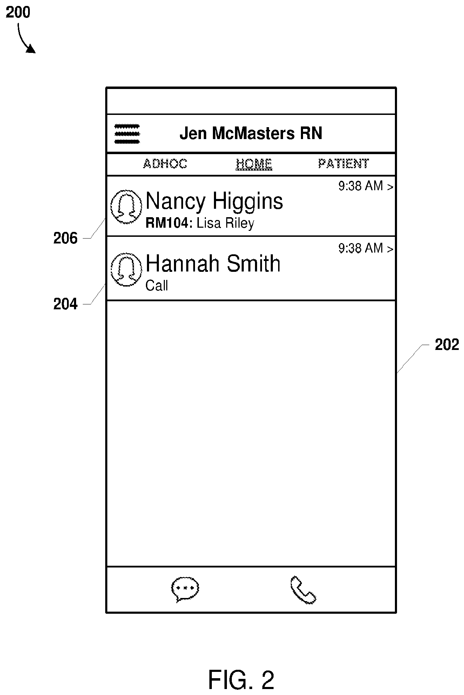

FIGS. 2-10 illustrate operations of a communication device 200 suitable for use in some embodiments. With reference to FIGS. 1-10, the illustrated operations may be implemented in hardware components and/or software components of the communication device (e.g., 102, 104), the operation of which may be controlled by one or more processors of the communication device (a "processor").

FIG. 2 illustrates a home screen 202 that may be displayed on the communication device 200. The home screen 202 may provide information such as a log or record of recent communication activity. For example, the home screen 202 may show a record of calls made or received, such as call log 204. As another example, the home screen 202 may show a log more detailed information, such as message log 206, showing a location of a communicating party ("Room 104") and a patient identity ("Lisa Riley") as well as a name of the communication party ("Nancy Higgins").

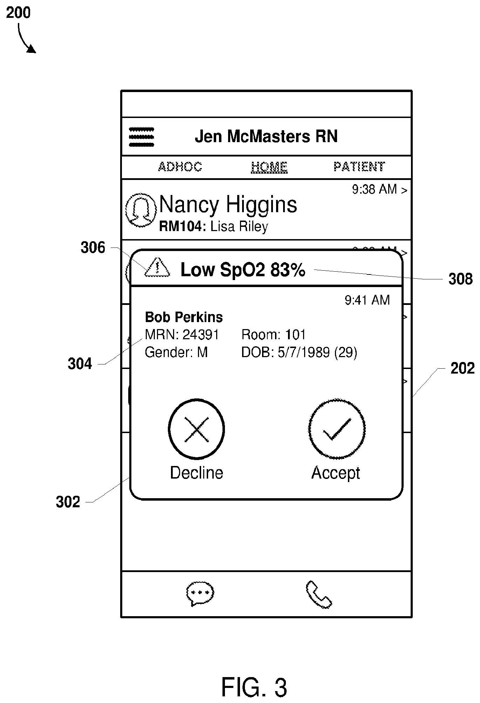

FIG. 3 illustrates an event alert 302 that may be displayed on the communication device 200. For example, the communication device 200 may be associated with a member of a patient's care team, and so may receive the event alert 302. In various embodiments, the communication device 200 may receive the event alert 302, an event identifier associated with the event alert 302, and information 304 related to the event alert 302. In some embodiments, the event alert 302 may be superimposed over other information (e.g., the other information displayed on home screen 202) for clarity and speed of communication. The event alert 302 may include an indication of the event 308 (e.g., that a patient has a low blood oxygen saturation level ("Low SpO2")) and other related information (e.g., the saturation level ("83%")). The event alert 302 may include patient information 304, such as a patient identity ("Bob Perkins"), a medical record number (MRN), a location of the patient, the patient's gender, date of birth, age, and other information. In some embodiments, the type, number, details, etc. of information provided in the event alert 302 may be configured as desired. In some embodiments, the related information 304 may be associated with the event identifier. The event alert 302 may include a priority indication 306 that indicates a level of urgency of the event alert, for example, an indication that the level of urgency is critical, high, medium, low, etc. For example, the illustrated indication 306 includes a symbol (e.g., an exclamation point in a triangle) indicating that this event alert is "urgent." In some embodiments, the indication may be graphical, color coded, audible, tactile, or another suitable indication. The event alert 302 may also present options for accepting the alert (i.e., indicating that the user will respond to or take responsibility for the alert) or declining the alert. The communication device 200 may receive an input (e.g., from a user) accepting or declining the alert.

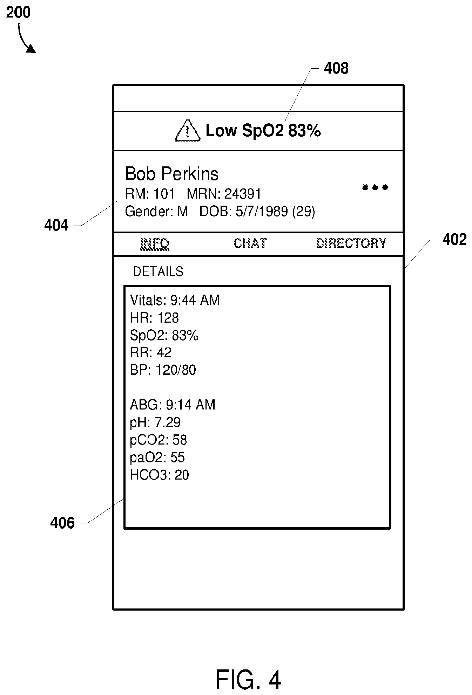

FIG. 4 illustrates an information screen 402 displayed on the communication device 200 after receiving an input accepting the event alert 302. The information screen 402 may include an indication of the event 408 (e.g., the accepted alert from the event alert 302) as well as patient information 404, 406. In some embodiments, the communication device 200 may display patient information 404, and may automatically display, or enable quick access to, additional patient information 406. In some embodiments, the patient information 404, 406 and the indication of the event 406 may be associated with an event identifier of the event 408. The patient information 404, 406 may, for example, include a variety of vital measurements of patient conditions, for example, heart rate (HR), SpO2, respiration rate (RR), blood pressure (BP), measurements of arterial blood gas (ABG), such as pH, partial pressure of carbon dioxide (pCO2), partial pressure of oxygen (paO2), bicarbonate level (HCO3), and the like. The information 404, 406 displayed may also include any type or variety of information, including environmental information, other patient information, and other suitable information.

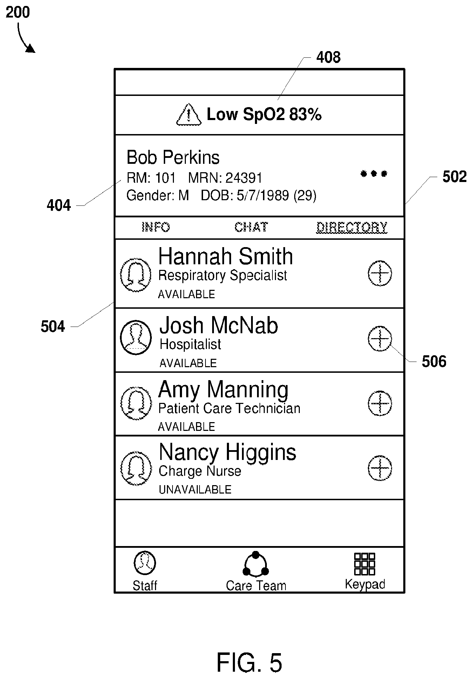

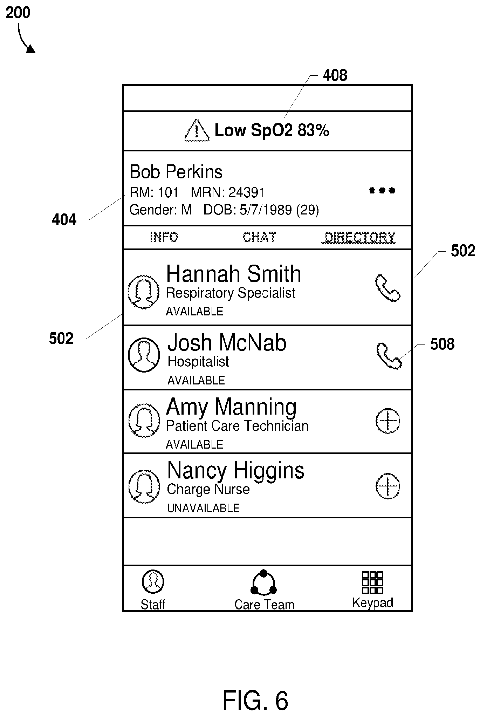

FIGS. 5 and 6 illustrate a directory screen 502 that may be displayed on the communication device 200. In response to receiving an input accepting the event alert 302, the communication device 200 may enable access to a directory 504 listing the care team members. The communication device 200 may also display the indication of the event 408 and or patient information 404. In some embodiments, the directory 504 and the indicator of the event 408 may be associated with the event identifier. In some embodiments, any communications initiated or received from the directory of care team members 504 may be associated with the event identifier.

In some embodiments, the directory 504 may include an indication of care team presence information indicating whether a communication device of the care team member is logged on to the communication network (e.g., "available," "unavailable," etc.). The directory 504 may also provide a function 506 to add care team members to a communication, such as a chat session, a voice communication session, or another suitable communication. The directory 504 may further provide an indication 508 (FIG. 6) that a care team member has been added to a communication. In some embodiments, the indication 508 enables the communication device 200 to initiate a voice communication with a communication device associated with a care team member.

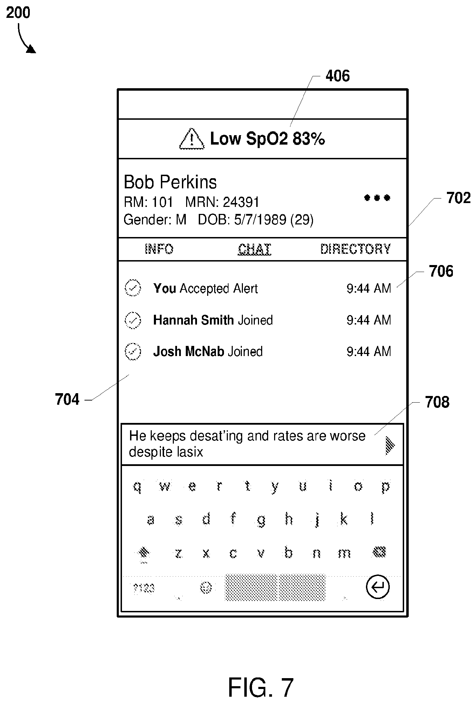

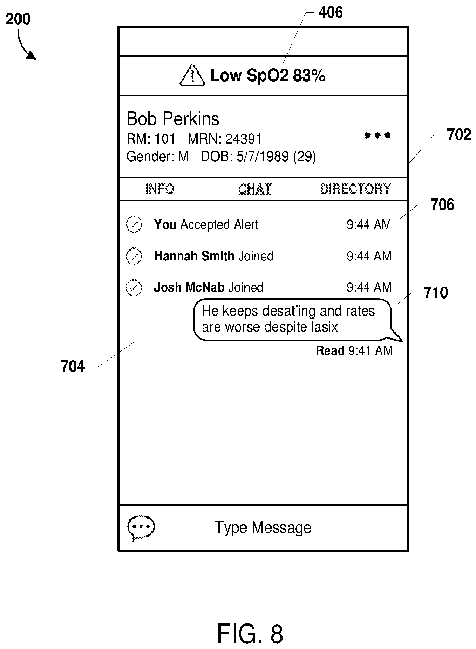

FIGS. 7 and 8 illustrate a chat screen 702 showing a chat function 704 supported by the user interface of a communication device 200 initiating a communication. The chat function 704 may include a log 706 of chat-related events, such as participants joining the chat session, messages sent or received (e.g., the message 708, 710), and other suitable items. The chat screen 702 may also display the indication of the event 406, which may be associated with an event identifier. In some embodiments, all communications (e.g., 708, 710) occurring within the chat function 704 may be associated with the event identifier.

In various embodiments, any of the communication devices participating in the chat may receive an input (e.g., a tap) to toggle among the information screen 402, the directory screen 502, and the chat screen702.

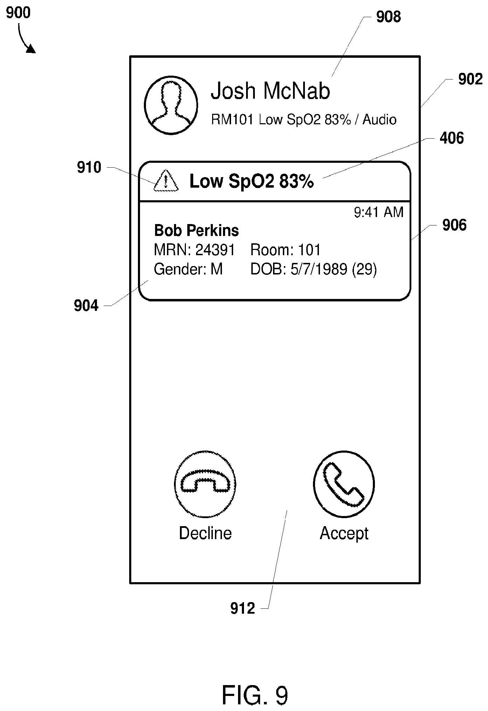

FIG. 9 illustrates a display of an incoming communication request 902 that may be displayed on a display device of a receiving communication device 900. In various embodiments, when the communication device 900 receives an incoming communication request (e.g., a call request), the communication device 900 presents the incoming communication request 902 to provide the user with call context information display 906 that enables the user to rapidly determine the context of the incoming call, for example, the subject of the call and relevant clinical information, before deciding whether to accept or decline the call. The call context information 906 may include, for example, the event identifier 406, patient information 904, caller information 908, and an indication of urgency 910 related to the communication request. In various embodiments, the call context information 902 and the elements 904, 906, 908, and 910 may each be associated with the event identifier.

The call context information display 906 may provide a detailed, information-rich notification of the subject matter of the communication request. In some embodiments, the call context information display 906 presents information that is clinically relevant to the communication request. For example, the call context information display 902 may include patient information 904, such as a patient identity, a medical record number, a location of the patient, the patient's gender, date of birth, age, and other information. The call context information display 902 may include an indication of an event 406 that is relevant to the communication request. The call context information display 902 may include an identifier of the caller 908 (i.e., the user of the communication device that is calling, such as the caller's name and location). The call context information display 906 may include a priority indication 910 that indicates a level of urgency of the communication request 902, or of the event associated with the communication request. For example, the priority indication 910 may indicate that the level of urgency is critical, high, medium, low, etc. In some embodiments, the indication may be graphical, color coded, audible, tactile, or another suitable indication. In some embodiments, the type, number, details, etc. of information provided in the call context information display 902 may be configured as desired. The communication request may also present graphical user interface (GUI) icons 912 for accepting the communication request (i.e., indicating that the user will participate in the requested communication) or declining the communication request 902. The receiving communication device 900 may receive a user input (e.g., by sensing a touch on one of the GUI icons 912) accepting or declining the communication request 902. In response to receiving a user input accepting the communication request 902, the communication device 900 may transmit messages to the wireless network to accept establishment of a communication session between the sending and receiving communication devices (e.g., by the voice server 130).

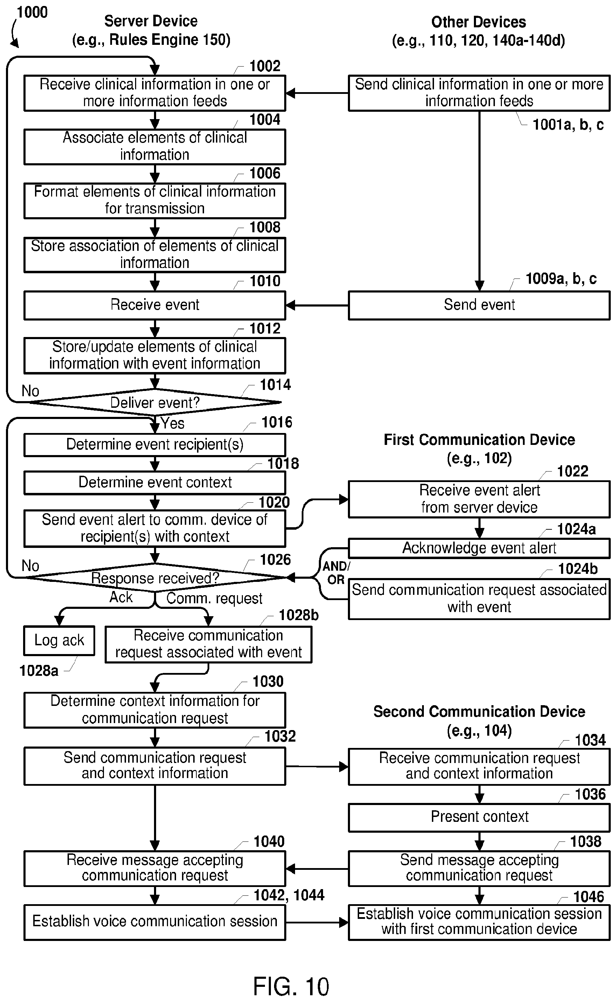

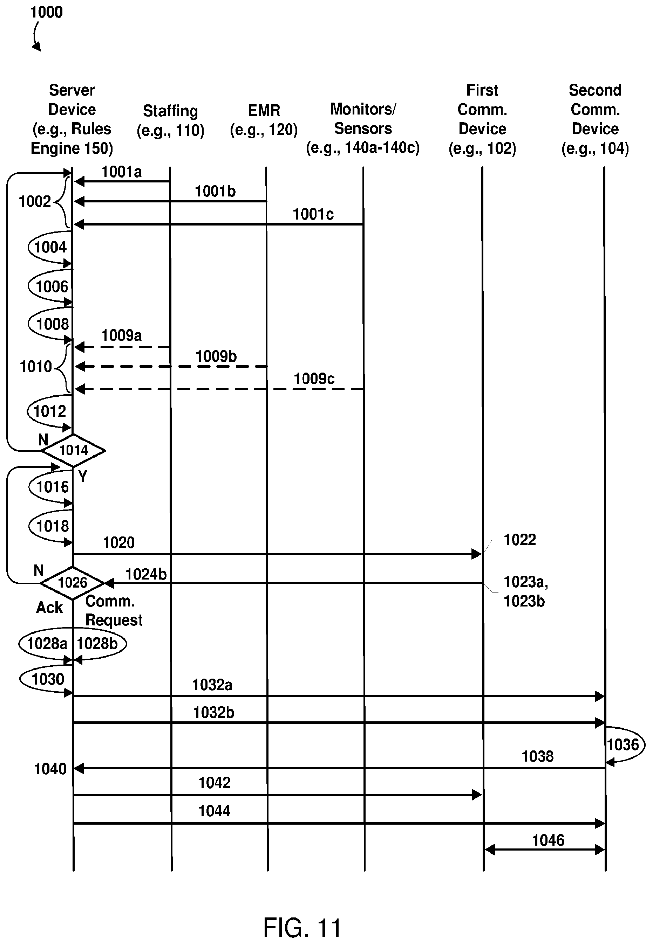

FIG. 10 illustrates a method 1000 and FIG. 11 illustrates signaling associated with the method 1000 for providing call context information along with a communication request to a communication device according to various embodiments. With reference to FIGS. 1-11, the method 1000 may be implemented in hardware components and/or software components of a rules engine (e.g., the rules engine 150) and/or a communication device (e.g., 102, 104), the operation of which may be controlled by one or more processors of the communication device (a "processor"). Some of the operations in the method 1000 may be performed in parallel and/or in an order different from that shown in FIGS. 10 and 11.

In operation 1001a, a staffing server (e.g., the staffing server 110) may send information in an information feed to the rules engine. In operation 1001b, an EMR server (e.g., the EMR server 120) may send information in an information feed to the rules engine. In operation 1001c, one or more sensors or data sources (e.g., the sensors/sources 4140a-140d) may send information in an information feed to the rules engine.

In operation 1002, the processor of the server device may receive clinical information in one or more information feeds from the one or more other devices (e.g., from the staffing server, the EMR server, and/or one or more sensor/sources.

In operation 1004, the processor of the server device may associate elements of the received clinical information. In some embodiments, the processor may parse the clinical information for one or more elements, and may associate the parsed elements of the clinical information. For example, the rules engine may associate information from one or more sensors/sources with a patient identifier and/or a location. As another example, patient identifiers and locations may be associated with each other based on EMR data (e.g., from the EMR server 120). As another example, staff assignment information (e.g., the staffing server 110) may be associated with a patient identifier and/or location.

In operation 1006, the processor of the server device may format the elements of the clinical information for transmission as call context information elements. In such operations, the processor may arrange, translate, transcode, or otherwise process the elements of clinical information into a format suitable for transmission, as well as condensing, summarizing and/or culling information so that inclusion in call context information will fit within the display of a communication device and be most useful to the recipient. For example, the processor may translate various vital signs into a compact format that a caregiver will understand.

In operation 1008, the processor of the server device may store the formatted clinical information (or information elements) in a database with one or more associations, such as links or index references.

In operation 1010, the processor of the server device may receive an event. For example, the processor of the server device may receive an event from the staffing server (1009a), the EMR server (1009b), one or more monitor/sensors (1009c), or from another network element or system. In some embodiments, an event includes a signal from a sensor or source (e.g., the sensors/sources 140a-140d) such as nurse call system, a patient physiological monitor, a video surveillance system, a bed exit monitor, or another suitable sensor or source of clinical information. In some embodiments, the event may include event identifying information, for example, event data (e.g., alarm data from a physiological monitor), an event type (e.g., an alarm type), an event time, and/or an event priority. In some embodiments, the event may include information indicating that one or more measurements are in range or out of range. For example, the event may include information indicating that a patient vital sign, an environmental condition, or the like is in or out of a normal or expected range. The event may include information indicating a patient identity or patient identifier and/or a location. In some embodiments, the event may include, or the processor of the server device may generate, an event identifier that identifies the event.

In operation 1012, the processor of the server device may store event information with the elements of the clinical information. In some embodiments, the processor of the server device may associate the received event with clinical information. For example, the processor may associate the event with a patient identifier and/or a location. In some embodiments, the processor may determine whether the event information includes an update to an existing event. In such embodiments, the processor may update the existing event information and associated clinical information with information from the updated event information. If the event is a new event, the processor may store the new event information and related clinical information. Event information and clinical information may be related in any number of ways, such as by patient identifier, by event location, by event type, event priority, event time, or other suitable relationship(s). In some embodiments, the processor may determine information to add or associate based on one or more relationships or correlations between or among event information and clinical information. For example, information from a monitor or sensor may be associated with a patient identity (or patient ID), and the processor may determine an event location based on the patient information associated with the monitor or sensor. As another example, the processor may determine staffing information based on EMR information and a patient identity. As another example, the processor may determine an event priority based on an event type and clinical information about a patient (e.g., a report of a low heart rate below a threshold for a patient in cardiac care may be determined as an urgent or high priority event). The processor may determine other information based on other suitable correlations.

**In** determination operation 1014, the processor of the server device may determine whether to send the event to a recipient. For example, the processor may determine whether to send the event to a recipient based on an event type, an event priority, an event time, a related patient identity or patient identifier, and/or an event location. In some embodiments, the processor may use one or more factors (e.g., event type, priority, time, patient ID, location, etc.) to determine a clinical relevance score. In such embodiments, the processor may compare the clinical relevance score to a relevance threshold, and may send the event to a recipient if the clinical relevance score exceeds the relevance threshold (or may not send the event if the clinical relevance score does not exceed the relevance threshold).

In response to determining that the event should not be sent to a recipient (i.e., determination operation 1014 = "No"), the processor of the server device may receive additional clinical information in one or more information feeds in operation 1002.

In response to determining that the event should be sent to a recipient (i.e., determination operation 1014 = "Yes"), the processor of the server device may determine one or more event recipients for the event in operation 1016. In some embodiments, the processor may determine a recipient based on event information, such as an event type, an event time, an event priority, a patient identity, and/or an event location. In some embodiments, the processor may be configured to determine a default, failsafe, or fallback recipient under certain conditions. For example, for an event lacking sufficient event information, clinical information, location information, a patient identity, or other information that may enable the processor to determine a recipient, the processor may identify a failsafe recipient to receive the event. For example, the processor may determine that a patient's lead physician is the failsafe recipient for an event. In various embodiments, the processor may use as much information as is available to determine a recipient where possible.

In operation 1018, the processor of the server device may determine an event context (i.e., context information) to send with the event. In some embodiments, the processor may determine information for the event context to send to a recipient with the event based on event information and/or clinical information. For example, the processor may determine the event context based on the event type, event time, event priority, patient identity, event location, patient status, clinical information, and/or other related information. In some embodiments, certain event types are considered sufficiently important to always include in the event context (e.g., need water, staff assist, asystole, priority, and location). In some embodiments, one or more aspects of the event may trigger the processor to obtain and/or include additional information and/or additional measurements in the event context. For example, if the event is a "LowSpO2" event, the processor may obtain a patient oxygen level (e.g., 83%) and include the patient oxygen level in the event context. Such additional information or measurements may include, for example, additional clinical information.

In operation 1020, the processor may send an event alert to a communication device of the identified one or more recipients. In various embodiments, the processor may send the event alert with the determined event context information.

In operation 1022, the processor of the communication device of the identified one or more recipients (i.e., a first communication device) may receive the event alert from the server device. In various embodiments, the processor of the first communication device may present the event context (e.g., on a display device) when providing a notification (e.g., an audible, visible, tactile, etc. notification) of the received event alert.

In some embodiments, in operation 1023a (FIG. 11), the processor of the first communication device may receive the event alert from the rules engine, and present a GUI display (e.g., 302) on the communication device user interface. The GUI display may include information from the event context and user input icons to permit the caregiver to accept, decline or otherwise acknowledge the event alert as illustrated in FIG. 3. In response to accepting the event alert, the processor may display more information regarding the event alert as illustrated in FIG. 4.

In operation 1024a, the processor of the communication device of the identified one or more recipients (i.e., a first communication device) may respond to the event alert by sending a message acknowledging the event alert.

Additionally or alternatively, in operation 1024b, the processor of the first communication device may respond to the event alert by sending a communication request associated with the event (e.g., associated via the event identifier). In some embodiments, in operation 1023b (FIG. 11), the processor of the first communication device may display a GUI interface to enable to caregiver to place a call to another in the care team (e.g., illustrated in FIG. 4).

In response to receiving a user input selecting a recipient for such a call, the processor may send a communication request to the rules engine in operation 1024b. In various embodiments, the communication request is associated with the event (e.g., by an event identifier or another suitable data element). In some embodiments, the communication request may be sent to a voice server (e.g., the voice server 130b), e.g., via a signaling gateway, and to the rules engine. In such embodiments, the voice server may perform various operations to establish a voice communication session with the recipient device with the event identifier, and the rules engine may determine context information, as further described below. In operation 1026, the processor of the server device may determine whether a response is been received from the first communication device. In response to determining that a response has not been received (i.e., determination operation = "No"), the processor of the server device may determine one or more additional event recipients in operation 1016.

In response to determining that an acknowledgment of the event alert has been received (i.e., determination operation = "Ack"), the processor of the server device may log the acknowledgment in operation 1028a. For example, the processor may store in a memory information indicating that the first communication device sent the acknowledgment of the event alert.

In response to determining that a communication request has been received (i.e., determination operation = "Comm. request"), the processor of the server device may receive the communication request associated with the event in operation 1028b.

In operation 1030, the processor of the server device may determine context information for the communication request. In some embodiments, based on the event and/or the intended recipient of the call request, the processor may determine the context information to send with the communication request. In some embodiments, the processor may determine the context information for the communication request based on a role of the intended recipient. For example, a call request to a physician may include clinically relevant information tailored to the physician's specialty. As another example, a call request to a physician's assistant or nurse may determine context information to include in the communication request based on the recipient's role. In some embodiments, the processor may use the same context determined and sent to the first communication device (e.g., operations 1020 and 1022). In some embodiments, based on the event, the processor may include additional information and/or obtain additional clinical information to include in the context. For example, a "Low SpO2" event may trigger the processor to include an oxygen saturation level (e.g., 83%), and may also trigger the processor to include additional clinical or other information. In some embodiments, the context information for the communication request to the second communication device may be different from the event context provided to the first communication device.

In operation 1032, the processor of the server device may send to the second communication device (e.g., the communication device 104) a communication request page or notification along with the context information. In some embodiments, the rules engine may provide the communication request and the context information to the second communication device in one signal or a series of signals. In some embodiments, the rules engine may provide the communication request and the context information in separate signaling, e.g., signals 1032a and 1032b. In some embodiments, the rules engine may provide the separate signaling in the same or different communication protocols or formats.

In operation 1034, a processor of the second communication device may receive the communication request and the context information from the rules engine.

In operation 1036, the processor of the second communication device may present the context information in a GUI display, such as illustrated in FIGS. 9 and 10. The GUI display may include user input icons to permit the recipient to accept or decline the communication request as illustrated in FIGS. 9 and 10.

In response to receiving a user input accepting the communication request, the processor of the second communication device may send a message to the rules engine accepting the communication request operation 1038. In some embodiments, the second communication device may send the message to the rules engine and to the voice server.

In operation 1040, the rules engine may receive the message accepting the communication request. In such embodiments, the voice server may also receive a message accepting the communication request, and may perform various operations to establish a voice communication session, as further described below.

In operations 1042 and 1044, the rules engine may send signaling 1042 to the first communication device and signaling 1044 to the second communication device as necessary to set up a voice communication session between the first communication device and the second communication device.

In operation 1046, the first communication device and the second communication device may conduct the voice communication session, which may be accomplished through the communication system 100.

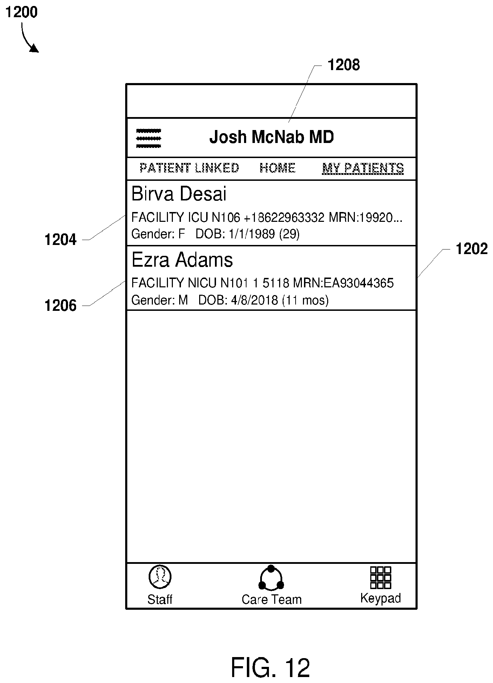

FIGS. 12-17 illustrate operations of a communication device 1200 suitable for use in some embodiments. With reference to FIGS. 1-12, the illustrated operations may be implemented in hardware components and/or software components of the communication device (e.g., 102, 104), the operation of which may be controlled by one or more processors of the communication device (a "processor").

FIG. 12 illustrates a My Patients screen 1202 that may be displayed on the communication device 1200. The My Patients screen 1202 may provide information, such as patient information (e.g., for patients "Birva Desai" and "Ezra Adams") assigned to or under the care of a user 1208 associated with the communication device 1200 (e.g., "Josh McNab"). For example, the My Patients screen 1202 may show patient indicators 1204, 1206 and certain information associated with each patient (e.g., facility or room where patient is located, gender of the patient, date of birth, or other information). In various embodiments, the information provided for each patient may be configured to display a variety of information as desired.

The My Patients screen 1202 may enable the communication device 1200 to initiate or request communication with another communication device about a patient. For example, the communication device 1200 may receive an input selecting patient Birva Desai.

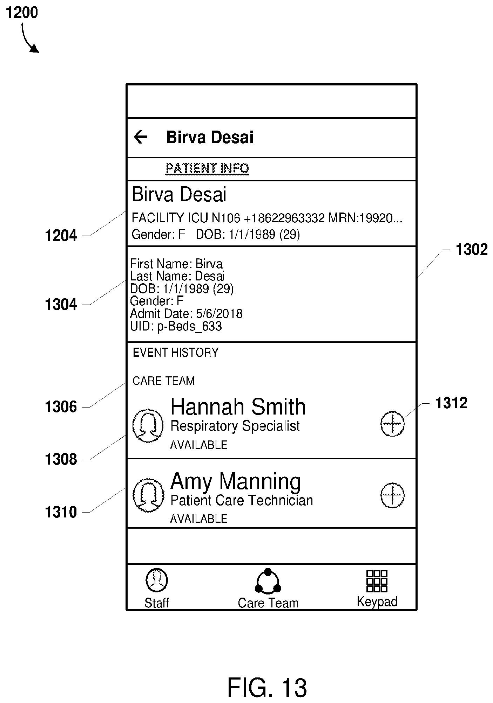

FIG. 13 illustrates a patient information screen 1302 that may be displayed on the communication device 1200 in response to receiving a selection of a patient on the My Patients screen 1202. In addition to the patient indicator 1204 for the selected patient, the patient information screen 1302 may also show additional patient information 1304. The patient information screen 1302 may also show a care team 1306 or other personnel 1308, 1310 associated with the patient (e.g., "Hannah Smith" and "Amy Manning"). The patient information screen 1302 may also provide a function 1312 to add care team members to a patient linked communication, such as a chat session, a voice communication session, or another suitable communication.

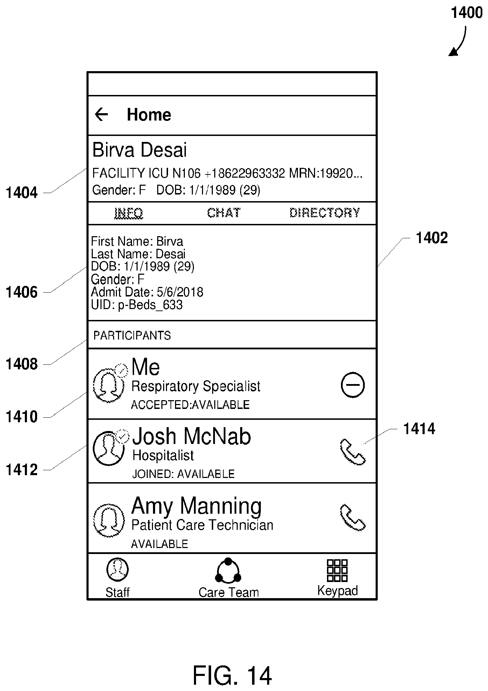

FIG. 14 illustrates an information screen 1402 that may be displayed on a second communication device 1400. In some embodiments, the second communication device 1400 may display the information screen 1402, or provide access to the information screen 1402, in response to receiving (or being joined or added to) the patient linked communication by the first communication device 1200. In this example, the second communication device 1400 is associated with a care team member that was added to a patient linked communication by the first communication device 1200 via function 1312 (FIG. 13) as described above. The information screen 1402 on the second communication device 1400 may show a patient indicator 1404 and associated patient information (which may be the same as the patient indicator 1204 and associated information displayed on the communication device 1200). The information screen 1402 may also show the additional patient information 1406 (which may be the same as the additional patient information 1302 displayed on the communication device 1200). The information screen 1404 may also show an indication of participants 1408 in the patient linked communication. For example, a participant indicator 1410 indicates that the user associated with the communication device 1400 is participating in the patient linked communication. Also, the participant indicator 1412 indicates that another user (e.g., "Josh McNab," associated with the communication device 1200 that initiated the patient linked communication) is also participating in the patient linked communication. In some embodiments, the indication 1414 may also enable the communication device 1400 to initiate a voice communication with a communication device associated with a care team member.

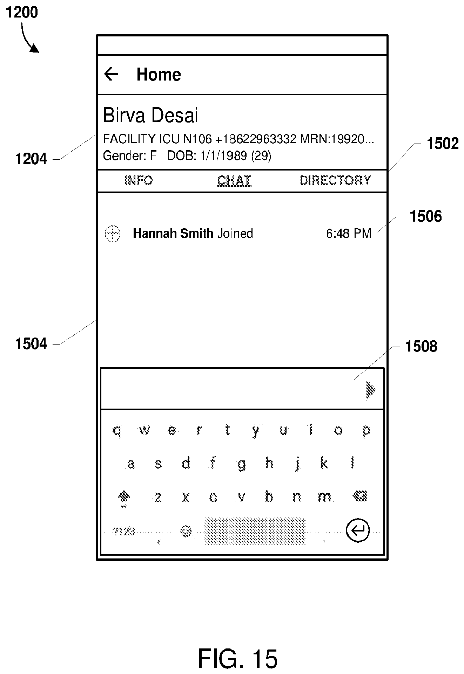

FIG. 15 illustrates a chat screen 1502 that may be displayed on the communication device 1200. The chat screen 1502 provides a chat function 1504 supported by the user interface of a communication device 1200 initiating a communication between the communication devices 1200 and 1400. The patient indicator 1204 provides an indication of the subject of the chat session (e.g., patient "Birva Desai"). The chat function 1504 may include a log 1506 of chat-related events, such as participants joining the chat session, messages sent or received, and other suitable items. In some embodiments, all chat-related events and communications occurring within the chat function 1504 may be associated with the patient (i.e., the subject of the chat session).

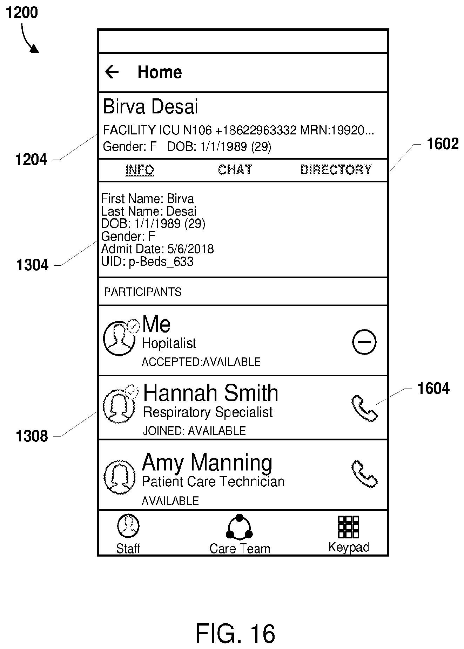

FIG. 16 illustrates an information screen 1602 that may be displayed on the communication device 1200. In addition to the patient indicator 1204 for the selected patient and the additional patient information 1304, the information screen 1602 may include a voice communication function 1604. In some embodiments, the voice communication function 1604 may be included or displayed with a particular care team member, such as care team member 1308 (e.g., "Hannah Smith" who is associated with the second communication device 1400). Activating the voice communication function 1604 may initiate a voice communication session from the communication device 1202 e.g., the second communication device 1400.

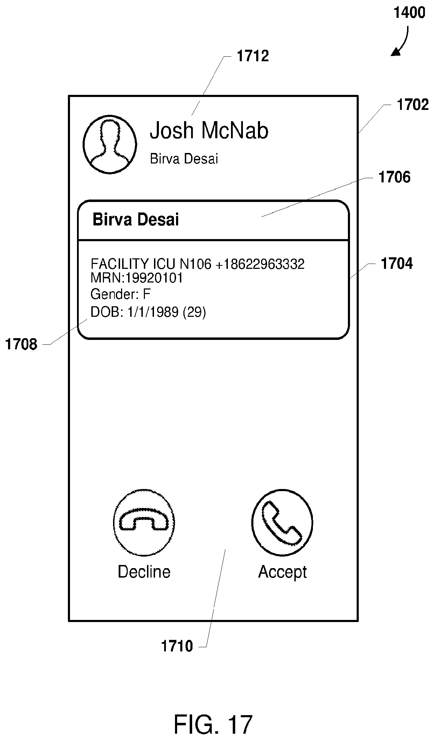

FIG. 17 illustrates a display of an incoming communication request 1702 that may be displayed on a receiving communication device (e.g., the second communication device 1400). In various embodiments, when the communication device 1400 receives a communication request, the communication device 1400 may display context information 1704 that provides context for the communication request. The context information 1704 may inform the recipient about the caller and provide context information that may be useful in deciding whether to accept the call. The context information 1704 may include a patient indicator 1706, as well as patient information 1708. In various embodiments, the context information 1704 and the elements 1706 and 1708 may be associated with a patient identifier. The incoming communication request 1702 may also include an indication 1712 identifying the initiator of the communication. In various embodiments, context information 1704 may provide a detailed, information-rich notification of the subject matter of the communication request. In some embodiments, the context information 1704 may present information that is clinically relevant to the communication request. For example, the patient information 1708 may include a patient identity, a medical record number, a location of the patient, the patient's gender, date of birth, age, and other information. In some embodiments, the type, number, details, etc. of information provided in the call context information 1704 may be configured as desired. The communication request 1702 may also present graphical user interface (GUI) icons 1710 for accepting the communication request (i.e., indicating that the user will participate in the requested communication) or declining the communication request. The receiving communication device 1400 may receive a user input (e.g., by sensing a touch on one of the GUI icons 1710) accepting or declining the communication request. In response to receiving a user input accepting the communication request, the communication device 1400 may transmit messages to the wireless network to accept establishment of a communication session between the sending and receiving communication devices (e.g., by the voice server 130).

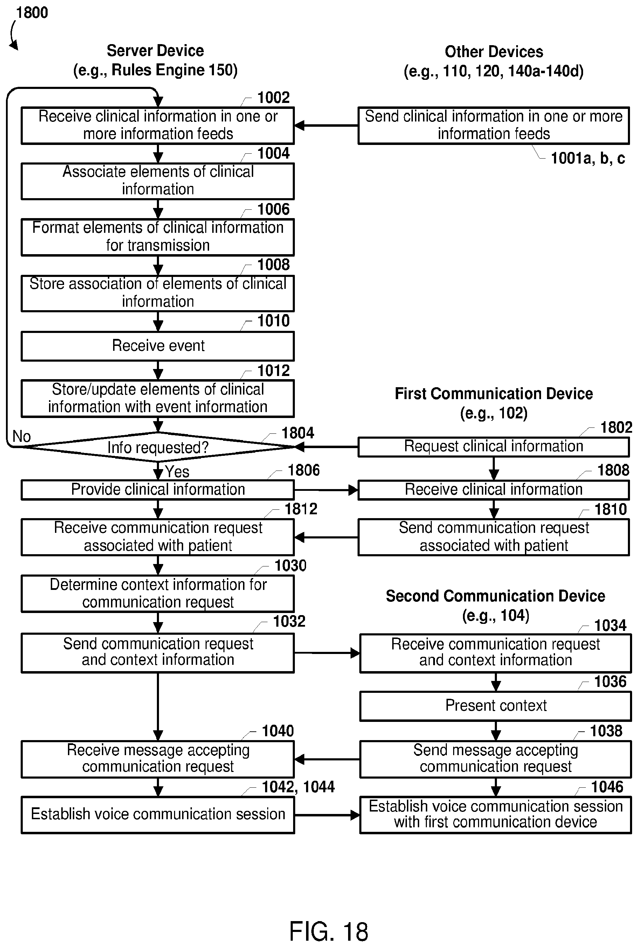

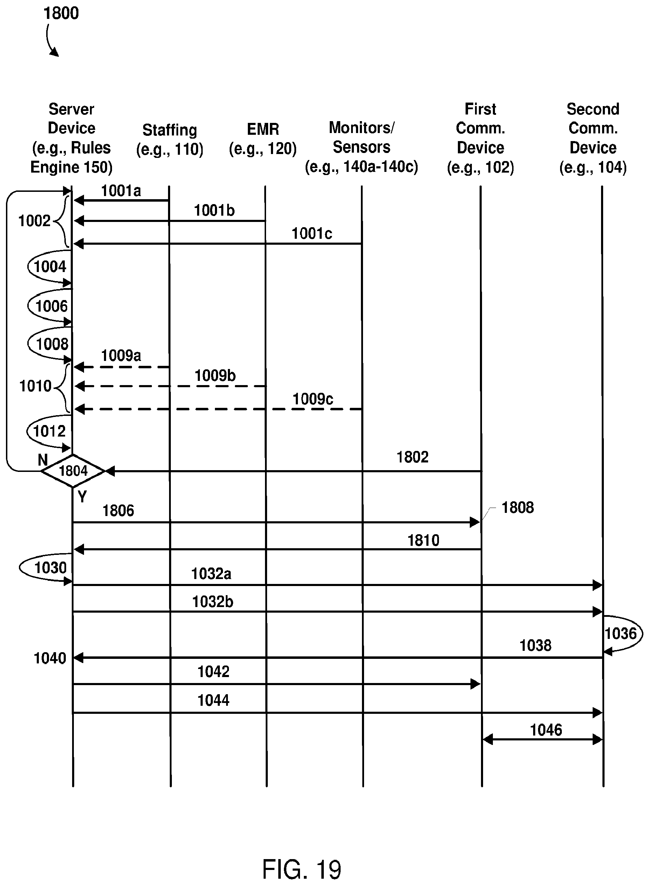

FIG. 18 illustrates a method 1800 and FIG. 19 illustrates signaling associated with the method 1800 for providing context information along with a communication request to a communication device according to various embodiments. With reference to FIGS. 1-19, the method 1800 may be implemented in hardware components and/or software components of a rules engine (e.g., the rules engine 150) and/or a communication device (e.g., 102, 104), the operation of which may be controlled by one or more processors of the communication device (a "processor"). Some of the operations in the method 1800 may be performed in parallel and/or in an order different from that shown in FIGS. 18 and 19. In operations 1102-1146, the processors of the server device, the first communication device, and the second communication device may perform operations of like-numbered operations of the method 1000 as described.

In operation 1802, the processor of the first communication device may request clinical information from the server device.

In determination operation 1804, the processor of the server device may determine whether information is been requested, such as by the first communication device (or another communication device). In response to determining that information has not been requested (i.e., determination operation 1804 = "No"), the server device processor may receive additional clinical information in operation 1102.

In response to determining that information has been requested (i.e., determination operation 1804 = "Yes"), the server device processor may provide clinical information to the requesting communication device in operation 1806.

In operation 1808, first communication device may receive the clinical information.

In operation 1810, the processor of the first communication device may send a communication request associated with a patient. In various embodiments, based on the received clinical information, the first communication device processor may send a patient linked communication request to the server device. In some embodiments, the patient linked communication request may include a request to initiate a communication with a second communication device, in which the communication request sent to the second communication device includes context information for the communication request. In some embodiments, context information may be associated with the patient who is the subject of the patient linked communication request.

In operation 1812, the processor of the server device may receive the communication request from the first communication device.

The processor of the server device may then determine a context for the information request in operation 1130, and send a communication request and the context to the second communication device in operation 1132, substantially as described.

By providing the call context information to the receiving communication device receiving a communication request, various embodiments improve the operation of the communication device and communication system overall by automatically providing an increased amount of relevant information (e.g., the clinically relevant information in the call context information) to the user of the receiving communication device, increasing the utility, efficacy, and speed of the communication system. Further, various embodiments increase the speed of communication of relevant information between parties even before a voice communication session is established between the parties. Moreover, various embodiments reduce the burden on caregivers by automatically compiling and sending information relevant to a communication request to the receiving communication device. Additionally, various embodiments improve the communication system by reducing the time required to convey relevant and/or time-sensitive information from one communication device to another.

FIG. 20 illustrates a communication device 2000 suitable for use in various embodiments. With reference to FIGS. 1-20, in some embodiments, the communication device 2000 may include a voice communications badge device. The communication device 2000 may include a housing 2002 that encloses various components. The communication device 2000 may include a microphone 2010, a speaker 2006, and a display device 2004 such as a liquid crystal display (LCD). Various information may be displayed on the display device 2004, such as data for reviewing text messages and pages received by the communication device 2000 and/or data to facilitate the operation of the communication device 2000. The microphone 2010 and speaker 2006 may also be used for voice communications. In some embodiments, the voice communication device 2000 may further include an amplifier that amplifies the signals provided to/from the microphone and speaker.

The communication device 2000 may further include an input device 2014 that permits a user to configure and operate the communication device 2000. In some embodiments, the input device 2014 may be a jog switch that may be a spring-loaded compound-action switch that supports three momentary actions. In such embodiments, the switch may be pressed inwards as an ordinary push button. In some embodiments, the input device 2014 may also be rotated in either direction and/or may be a touch button location in particular location (e.g., on the front of the communication device 2000) that may be pushed or touched to activate the same functions and operations being activated by the jog switch. The communication device 2000 may also include an on/off switch 2016 and a status indicator (e.g., an LED that may be capable of displaying one or more different colors to signal the operational status of the communication device 2000, etc.). In some embodiments, the communication device 2000 may optionally include a headset jack that enables the user to plug in an external microphone/ speaker headset, such as an ear bud.

Internally, the communication device 2000 may include a central processing unit (CPU) or processor 2050 that controls the operation of the components of the communication device 2000. For example, the processor 2050 may control the operations of the microphone 2010 and the speaker 2006 so that the communication device 2000 may exchange voice communications, commands, and/or responses with remote devices (e.g., a voice communications server, etc.). The communication device 2000 may further include a non-volatile memory device 2052 so that data stored in the communication device 2000 (such as settings, messages, and other data structures) are not lost when the communication device 2000 is powered down. For example, the non-volatile memory device 2052 may be a storage unit or other memory device configured to store at least a factory-assigned a unique physical media access control (MAC) address or unique wireless device address. The communication device 2000 may also include a wireless transceiver 2054 (e.g., an appropriate strength 802.11 transceiver, etc.) and an antenna 2056 that may be used for wireless communications with various access points or with other devices (e.g., other communication devices, etc.). In some embodiments, the antennae 2056 may be built into an exterior clip of the communication device 2000 or may reside completely within the housing 802 of the communication device 2000.

The communication device 2000 may further include a pager receiver 2060 that operates with the antenna 2056 to receive text messages/pages within the coverage of any global paging service network. The communication device 2000 may further comprise a digital signal processor (DSP) 2062 and an audio codec 2064 for processing incoming speech from the microphone 810 and for generating the voice signals generated by the speaker 2006. For example, the DSP 2062 and audio codec 2064 may be capable of compressing digital voice data to reduce the amount of digital data used to communicate the voice commands to the server. The communication device 2000 may include a power source 2058, such as a removable, rechargeable battery that may include protection and charge management circuitry to prevent overcharging. For example, the energy source 2058 may be a replaceable, rechargeable lithium polymer or lithium ion battery that fits on or in the housing 2002. The various components may be connected via a bus or other similar linkage or connectivity.

Exemplary descriptions of various voice communications badge devices suitable for use in various embodiments may also be found in commonly-held patent applications, including U.S. Patent 6,892,083 entitled "Voice-Controlled Wireless Communications System and Method," U.S. Patent 8,098,806 entitled "Non-User-Specific Wireless Communication System and Method," and U.S. Design Patent D679,673.

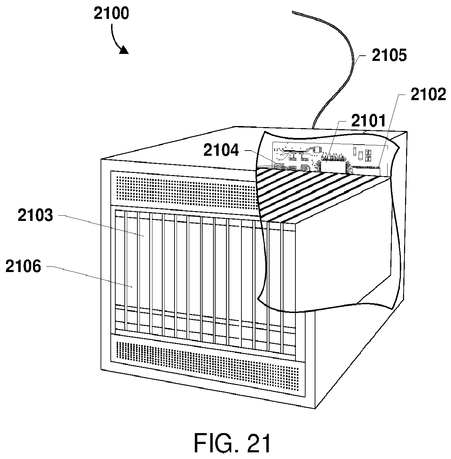

FIG. 21 illustrates a server device 2100 suitable for use in various embodiments. With reference to FIGS. 1-21, various embodiments may employ the server device 2100 as a network element of a communication system (e.g., the communication system 100). Examples of network elements that may be implemented in a server device, or as a logical service in a server device, include the staffing server 110, the EMR server 120, the messaging server 130a, the voice communications server 130b, and the rules engine 150. The server device 2100 may include a processor 2101 coupled to volatile memory 2102 and a large capacity nonvolatile memory, such as a disk drive 2103. The server device 2100 may also include a peripheral memory access device such as a floppy disc drive, compact disc (CD) or digital video disc (DVD) drive 2106 coupled to the processor 2101. The server device 2100 may also include network access ports 2104 (or interfaces) coupled to the processor 2101 for establishing data connections with a network, such as the Internet and/or a local area network coupled to other system computers and servers. The server device 2100 may include one or more antennas 2107 for sending and receiving electromagnetic radiation that may be connected to a wireless communication link. The server device 2100 may include additional access ports, such as USB, Firewire, Thunderbolt, and the like for coupling to peripherals, external memory, or other devices.

The various processors described herein may be any programmable microprocessor, microcomputer or multiple processor chip or chips that can be configured by software instructions (applications) to perform a variety of functions, including the functions of the various embodiments described herein. In the various devices, multiple processors may be provided, such as one processor dedicated to wireless communication functions and one processor dedicated to running other applications. Typically, software applications may be stored in internal memory before they are accessed and loaded into the processors. The processors may include internal memory sufficient to store the application software instructions. In many devices the internal memory may be a volatile or nonvolatile memory, such as flash memory, or a mixture of both. For the purposes of this description, a general reference to memory refers to memory accessible by the processors including internal memory or removable memory plugged into the various devices and memory within the processors.

The foregoing method descriptions and the process flow diagrams are provided merely as illustrative examples and are not intended to require or imply that the operations of the various embodiments must be performed in the order presented. Accordingly, the order of operations in the foregoing embodiments may be performed in any order. Words such as "thereafter," "then," "next," etc. are not intended to limit the order of the operations; these words are simply used to guide the reader through the description of the methods. Further, any reference to claim elements in the singular, for example, using the articles "a," "an" or "the" is not to be construed as limiting the element to the singular.

The various illustrative logical blocks, modules, circuits, and algorithm operations described in connection with the embodiments disclosed herein may be implemented as electronic hardware, computer software, or combinations of both. To clearly illustrate this interchangeability of hardware and software, various illustrative components, blocks, modules, circuits, and operations have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

The hardware used to implement the various illustrative logics, logical operations, modules, and circuits described in connection with the embodiments disclosed herein may be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but, in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration. Alternatively, some operations or methods may be performed by circuitry that is specific to a given function.

In one or more exemplary embodiments, the functions described may be implemented in hardware, software, firmware, or any combination thereof. If implemented in software, the functions may be stored on or transmitted over as one or more instructions or code on a non-transitory processor-readable, computer-readable, or server-readable medium or a non-transitory processor-readable storage medium. The operations of a method or algorithm disclosed herein may be embodied in a processor-executable software module or processor-executable software instructions which may reside on a non-transitory computer-readable storage medium, a non-transitory server-readable storage medium, and/or a non-transitory processor-readable storage medium. In various embodiments, such instructions may be stored processor-executable instructions or stored processor-executable software instructions. Tangible, non-transitory computer-readable storage media may be any available media that may be accessed by a computer. By way of example, and not limitation, such non-transitory computer-readable media may comprise RAM, ROM, EEPROM, CD-ROM or other optical disk storage, magnetic disk storage or other magnetic storage devices, or any other medium that may be used to store desired program code in the form of instructions or data structures and that may be accessed by a computer. Disk and disc, as used herein, includes compact disc (CD), laser disc, optical disc, digital versatile disc (DVD), floppy disk, and Blu-ray disc^{®} where disks usually reproduce data magnetically, while discs reproduce data optically with lasers. Combinations of the above should also be included within the scope of non-transitory computer-readable media. Additionally, the operations of a method or algorithm may reside as one or any combination or set of codes and/or instructions on a tangible, non-transitory processor-readable storage medium and/or computer-readable medium, which may be incorporated into a computer program product.

The preceding description of the disclosed embodiments is provided to enable any person skilled in the art to make or use the present invention. Thus, the present invention is not intended to be limited to the embodiments shown herein but is to be accorded the widest scope consistent with the following claims and the principles and novel features disclosed herein.

## Claims

1. A method of providing context information in a medical communication system, comprising:
receiving, by a server device, clinical information in one or more information feeds from one or more other devices;
determining, by the server device, whether a communication request from a first communication device for a second communication device has been received;
determining, by the server device, an event identifier associated with the communication request from the first communication device;
selecting, by the server device from among the received clinical information, elements of the clinical information associated with the event identifier to provide as call context information to the second communication device, wherein the second communication device is associated with a user having a role, and wherein the selecting of the elements of the clinical information to provide as call context information to the second communication device depends on the role of the user associated with the second communication device;
sending, by the server device to the second communication device, a communication request message to initiate a voice communication session in response to the communication request from the first communication device; and
based on the selecting, sending by the server device to the second communication device, along with the communication request message, call context information including the selected elements of the clinical information, to enable the second communication device to present the selected elements of the clinical information as a supplement to caller-identification information for the voice communication session.

2. The method of claim 1, further comprising:
associating, by the server device, clinically relevant elements of the clinical information with one or more event identifiers.

3. The method of claim 2, further comprising:
formatting, by the server device, the elements of clinical information into a format for transmission as part of the call context information to the second communication device.

4. The method of claim 2, further comprising:
storing, by the server device, the association of the elements of clinical information and the one or more event identifiers in a database.

5. The method of claim 1, further comprising:
determining whether a patient event alert has been received or should be issued based upon the received clinical information; and
sending, by the server device, to the first communication device an event alert in response to determining that a patient event alert has been received or should be issued, the event alert including the event identifier and event context information.

6. The method of claim 5, further comprising:
receiving, by the first communication device, the event alert;
displaying, by the first communication device, a graphical user interface (GUI) display identifying the event identifier and the event context information along with user interface icons for accepting or declining the event alert;
displaying, by the first communication device, a GUI display to enable a caregiver to place a communication request related to the event alert; and
transmitting, by the first communication device, to the server device the communication request, as a request related to the event alert, in response to receiving a user input to initiate the voice communication session.

7. The method of claim 1, further comprising:
receiving, by the second communication device, the communication request message along with the call context information including the selected elements of the clinical information;
displaying, by the second communication device, a graphical user interface (GUI) display including an event identifier and the call context information along with user interface icons for accepting or declining the voice communication session; and
transmitting, by the second communication device, to the server device a message to accept the voice communication session in response to receiving a user input to accept the voice communication session.

8. The method of claim 1, wherein sending by the server device to the second communication device, along with the communication request message, the call context information including the selected elements of the clinical information comprises sending to the second communication device, in one signal, the communication request message and the call context information.

9. A server device, comprising:
a processor configured with processor-executable instructions to:
receive clinical information in one or more information feeds from one or more other devices;
determine whether when a communication request from a first communication device for a second communication device has been received;
determine an event identifier associated with the communication request from the first communication device;
select from among the received clinical information elements of the clinical information associated with the event identifier to provide as call context information to the second communication device, wherein the second communication device is associated with a user having a role, and wherein the selecting of the elements of the clinical information to provide as call context information to the second communication device depends on the role of the user associated with the second communication device;
send a communication request message to the second communication device, a communication request message to initiate a voice communication session in response to the communication request from the first communication device; and
based on the selecting, send to the second communication device, along with the communication request, call context information including the selected elements of the clinical information, to enable the second communication device to present the selected elements of the clinical information as a supplement to caller-identification information for the voice communication session.

10. The server device of claim 9, wherein the processor is further configured with processor-executable instructions to:
associate clinically relevant elements of the clinical information with one or more event identifiers.

11. The server device of claim 10, wherein the processor is further configured with processor-executable instructions to:
format the elements of clinical information into a format for transmission as part of the call context information to the second communication device.

12. The server device of claim 10, wherein the processor is further configured with processor-executable instructions to:
store the association of the elements of clinical information and the one or more event identifiers in a database.

13. The server device of claim 12, wherein the processor is further configured with processor-executable instructions to:
determine whether a patient event alert has been received or should be issued based upon the received clinical information; and
send to the first communication device an event alert in response to determining that a patient event alert has been received or should be issued, the event alert including the event identifier and event context information.

14. The server device of claim 9, wherein sending to the second communication device, along with the communication request message, the call context information including the selected elements of the clinical information comprises sending to the second communication device, in one signal, the communication request message and the call context information.

15. A system, comprising:
a server device;
a first communication device; and
a second communication device,
wherein the server device comprises a server processor configured with processor-executable instructions to:
receive clinical information in one or more information feeds from one or more other devices;
determine whether when a communication request from the first communication device for the second communication device has been received;
determine an event identifier associated with the communication request from the first communication device;
select from among the received clinical information elements of the clinical information associated with the event identifier to provide as call context information to the second communication device, wherein the second communication device is associated with a user having a role, and wherein the selecting of the elements of the clinical information to provide as call context information to the second communication device depends on the role of the user associated with the second communication device;
send to the second communication device, a communication request message to initiate a voice communication session in response to the communication request from the first communication devices; and
based on the selecting, send to the second communication device, along with the second communication request, call context information including the selected elements of the clinical information, to enable the second communication device to present the selected elements of the clinical information as a supplement to caller-identification information for the voice communication session;
wherein the first communication device comprises a first device processor configured with processor-executable instructions to:
receive an event alert;
display a graphical user interface (GUI) display including an event identifier and event context information included in the event alert along with user interface icons for accepting or declining the event alert;
display a GUI display to enable a caregiver to place a communication request related to the event alert; and
transmit to the server device the communication request, as a request related to the event alert, in response to receiving a user input to initiate the voice communication session; and
wherein the second communication device comprises a second device processor configured with processor-executable instructions to:
receive from the server device the communication request message along with the call context information including the selected elements of the clinical information;
display a GUI display including an event identifier and the call context information along with user interface icons for accepting or declining the voice communication session; and
transmit to the server device a message to accept the voice communication session in response to receiving a user input to accept the voice communication session.

16. The system of claim 15, wherein the server processor is further configured with processor-executable instructions to:
associate clinically relevant elements of the clinical information with one or more event identifiers.

17. The system of claim 16, wherein the server processor is further configured with processor-executable instructions to:
format the elements of clinical information into a format for transmission as part of the call context information to the second communication device.

18. The system of claim 16, wherein the server processor is further configured with processor-executable instructions to:
store the association of the elements of clinical information and the one or more event identifiers in a database.

19. The system of claim 15, wherein the server processor is further configured with processor-executable instructions to:
determine whether a patient event alert has been received or should be issued based upon the received clinical information; and
send to the first communication device an event alert in response to determining that a patient event alert has been received or should be issued, the event alert including the event identifier and the event context information.

20. The system of claim 15, wherein sending to the second communication device, along with the communication request message, the call context information including the selected elements of the clinical information comprises sending to the second communication device, in one signal, the communication request message and the call context information.

21. A non-transitory processor readable storage medium having stored thereon processor-executable instructions configured to cause a processor of a server device to perform operations comprising the steps of any one of claims 1 to 5 and 8.

## Patentansprüche

1. Verfahren zum Bereitstellen von Kontextinformationen in einem medizinischen Kommunikationssystem, umfassend:
Empfangen, durch eine Servervorrichtung, von klinischen Informationen in einem oder mehreren Informationsfeeds von einer oder mehreren anderen Vorrichtungen;
Bestimmen, durch die Servervorrichtung, ob eine Kommunikationsanforderung von einer ersten Kommunikationsvorrichtung für eine zweite Kommunikationsvorrichtung empfangen wurde;
Bestimmen, durch die Servervorrichtung, eines Ereignisidentifizierers, der der Kommunikationsanforderung von der ersten Kommunikationsvorrichtung zugeordnet ist;
Auswählen, durch die Servervorrichtung aus den empfangenen klinischen Informationen, von Elementen der klinischen Informationen, die dem Ereignisidentifizierer zugeordnet sind, um diese als Anrufkontextinformationen an die zweite Kommunikationsvorrichtung bereitzustellen, wobei die zweite Kommunikationsvorrichtung einem Benutzer zugeordnet ist, der eine Rolle aufweist, und wobei das Auswählen der Elemente der klinischen Informationen, die als Anrufkontextinformationen an die zweite Kommunikationsvorrichtung bereitzustellen sind, von der Rolle des Benutzers abhängt, der der zweiten Kommunikationsvorrichtung zugeordnet ist;
Senden, durch die Servervorrichtung an die zweite Kommunikationsvorrichtung, einer Kommunikationsanforderungsnachricht, um eine Sprachkommunikationssitzung als Reaktion auf die Kommunikationsanforderung von der ersten Kommunikationsvorrichtung zu initiieren; und
basierend auf dem Auswählen, Senden durch die Servervorrichtung an die zweite Kommunikationsvorrichtung, zusammen mit der Kommunikationsanforderungsnachricht, von Anrufkontextinformationen, die die ausgewählten Elemente der klinischen Informationen einschließen, um der zweiten Kommunikationsvorrichtung zu ermöglichen, die ausgewählten Elemente der klinischen Informationen als eine Ergänzung zu Anruferidentifikationsinformationen für die Sprachkommunikationssitzung darzustellen.

2. Verfahren nach Anspruch 1, ferner umfassend:
Zuordnen, durch die Servervorrichtung, klinisch relevanter Elemente der klinischen Informationen zu einem oder mehreren Ereignisidentifizierern.

3. Verfahren nach Anspruch 2, ferner umfassend:
Formatieren, durch die Servervorrichtung, der Elemente klinischer Informationen in ein Format für eine Übertragung als Teil der Anrufkontextinformationen an die zweite Kommunikationsvorrichtung.

4. Verfahren nach Anspruch 2, ferner umfassend:
Speichern, durch die Servervorrichtung, der Zuordnung der Elemente klinischer Informationen und der einen oder der mehreren Ereignisidentifizierern in einer Datenbank.

5. Verfahren nach Anspruch 1, ferner umfassend:
Bestimmen, ob eine Patientenereigniswarnung empfangen wurde oder ausgegeben werden sollte, basierend auf den empfangenen klinischen Informationen; und
Senden, durch die Servervorrichtung, an die erste Kommunikationsvorrichtung einer Ereigniswarnung als Reaktion auf das Bestimmen, dass eine Patientenereigniswarnung empfangen wurde oder ausgegeben werden sollte, wobei die Ereigniswarnung den Ereignisidentifizierer und Ereigniskontextinformationen einschließt.

6. Verfahren nach Anspruch 5, ferner umfassend:
Empfangen, durch die erste Kommunikationsvorrichtung, der Ereigniswarnung;
Anzeigen, durch die erste Kommunikationsvorrichtung, einer grafischen Benutzerschnittstellenanzeige (GUI-Anzeige), die den Ereignisidentifizierer und die Ereigniskontextinformationen zusammen mit Benutzerschnittstellensymbolen zum Annehmen oder Ablehnen der Ereigniswarnung identifiziert;
Anzeigen, durch die erste Kommunikationsvorrichtung, einer GUI-Anzeige, um es einer Pflegekraft zu ermöglichen, eine Kommunikationsanforderung bezüglich der Ereigniswarnung zu stellen; und
Übertragen, durch die erste Kommunikationsvorrichtung, an die Servervorrichtung der Kommunikationsanforderung, als eine Anforderung bezüglich der Ereigniswarnung als Reaktion auf das Empfangen einer Benutzereingabe, um die Sprachkommunikationssitzung zu initiieren.

7. Verfahren nach Anspruch 1, ferner umfassend:
Empfangen, durch die zweite Kommunikationsvorrichtung, der Kommunikationsanforderungsnachricht zusammen mit den Anrufkontextinformationen, die die ausgewählten Elemente der klinischen Informationen einschließen;
Anzeigen, durch die zweite Kommunikationsvorrichtung, einer grafischen Benutzerschnittstellenanzeige (GUI-Anzeige), die einen Ereignisidentifizierer und die Anrufkontextinformationen zusammen mit Benutzerschnittstellensymbolen zum Annehmen oder Ablehnen der Sprachkommunikationssitzung einschließt; und
Übertragen, durch die zweite Kommunikationsvorrichtung, an die Servervorrichtung einer Nachricht, um die Sprachkommunikationssitzung als Reaktion auf das Empfangen einer Benutzereingabe anzunehmen, um die Sprachkommunikationssitzung anzunehmen.

8. Verfahren nach Anspruch 1, wobei das Senden durch die Servervorrichtung an die zweite Kommunikationsvorrichtung, zusammen mit der Kommunikationsanforderungsnachricht, der Anrufkontextinformationen, die die ausgewählten Elemente der klinischen Informationen einschließen, das Senden an die zweite Kommunikationsvorrichtung, in einem Signal, der Kommunikationsanforderungsnachricht und der Anrufkontextinformationen umfasst.

9. Servervorrichtung, umfassend:
ein Prozessor, der mit prozessorausführbaren Anweisungen konfiguriert ist zum:
Empfangen klinischer Informationen in einem oder mehreren Informationsfeeds von einem oder mehreren anderen Vorrichtungen;
Bestimmen, ob eine Kommunikationsanforderung von einer ersten Kommunikationsvorrichtung für eine zweite Kommunikationsvorrichtung empfangen wurde;
Bestimmen eines Ereignisidentifizierers, der der Kommunikationsanforderung von der ersten Kommunikationsvorrichtung zugeordnet ist;
Auswählen aus den empfangenen klinischen Informationselementen der klinischen Informationen, die dem Ereignisidentifizierer zugeordnet sind, um diese als Anrufkontextinformationen an die zweite Kommunikationsvorrichtung bereitzustellen, wobei die zweite Kommunikationsvorrichtung einem Benutzer zugeordnet ist, der eine Rolle aufweist, und wobei das Auswählen der Elemente der klinischen Informationen, die als Anrufkontextinformationen an die zweite Kommunikationsvorrichtung bereitzustellen sind, von der Rolle des Benutzers abhängt, der der zweiten Kommunikationsvorrichtung zugeordnet ist;
Senden einer Kommunikationsanforderungsnachricht an die zweite Kommunikationsvorrichtung, eine Kommunikationsanforderungsnachricht, um eine Sprachkommunikationssitzung als Reaktion auf die Kommunikationsanforderung von der ersten Kommunikationsvorrichtung zu initiieren; und
basierend auf dem Auswählen, Senden an die zweite Kommunikationsvorrichtung, zusammen mit der Kommunikationsanforderung, von Anrufkontextinformationen, die die ausgewählten Elemente der klinischen Informationen einschließen, um der zweiten Kommunikationsvorrichtung zu ermöglichen, die ausgewählten Elemente der klinischen Informationen als eine Ergänzung zu Anruferidentifikationsinformationen für die Sprachkommunikationssitzung darzustellen.

10. Servervorrichtung nach Anspruch 9, wobei der Prozessor ferner mit prozessorausführbaren Anweisungen konfiguriert ist zum:
Zuordnen klinisch relevanter Elemente der klinischen Informationen mit einem oder mehreren Ereignisidentifizierern.

11. Servervorrichtung nach Anspruch 10, wobei der Prozessor ferner mit prozessorausführbaren Anweisungen konfiguriert ist zum:
Formatieren der Elemente klinischer Informationen in ein Format für die Übertragung als Teil der Anrufkontextinformationen an die zweite Kommunikationsvorrichtung.

12. Servervorrichtung nach Anspruch 10, wobei der Prozessor ferner mit prozessorausführbaren Anweisungen konfiguriert ist zum:
Speichern der Zuordnung der Elemente klinischer Informationen und des einen oder der mehreren Ereignisidentifizierern in einer Datenbank.

13. Servervorrichtung nach Anspruch 12, wobei der Prozessor ferner mit prozessorausführbaren Anweisungen konfiguriert ist zum:
Bestimmen, ob eine Patientenereigniswarnung empfangen wurde oder basierend auf den empfangenen klinischen Informationen ausgegeben werden sollte; und
Senden an die erste Kommunikationsvorrichtung einer Ereigniswarnung als Reaktion auf das Bestimmen, dass eine Patientenereigniswarnung empfangen wurde oder ausgegeben werden sollte, wobei die Ereigniswarnung den Ereignisidentifizierer und Ereigniskontextinformationen einschließt.

14. Servervorrichtung nach Anspruch 9, wobei das Senden an die zweite Kommunikationsvorrichtung, zusammen mit der Kommunikationsanforderungsnachricht, der Anrufkontextinformationen, die die ausgewählten Elemente der klinischen Informationen einschließen, das Senden an die zweite Kommunikationsvorrichtung, in einem Signal, der Kommunikationsanforderungsnachricht und der Anrufkontextinformationen umfasst.

15. System, umfassend:
eine Servervorrichtung;
eine erste Kommunikationsvorrichtung; und
eine zweite Kommunikationsvorrichtung,
wobei die Servervorrichtung einen Serverprozessor umfasst, der mit prozessorausführbaren Anweisungen konfiguriert ist zum:
Empfangen klinischer Informationen in einem oder mehreren Informationsfeeds von einem oder mehreren anderen Vorrichtungen;
Bestimmen, ob, wenn eine Kommunikationsanforderung von der ersten Kommunikationsvorrichtung für die zweite Kommunikationsvorrichtung empfangen wurde;
Bestimmen eines Ereignisidentifizierers, der der Kommunikationsanforderung von der ersten Kommunikationsvorrichtung zugeordnet ist;
Auswählen aus den empfangenen klinischen Informationselementen der klinischen Informationen, die dem Ereignisidentifizierer zugeordnet sind, um diese als Anrufkontextinformationen an die zweite Kommunikationsvorrichtung bereitzustellen, wobei die zweite Kommunikationsvorrichtung einem Benutzer zugeordnet ist, der eine Rolle aufweist, und wobei das Auswählen der Elemente der klinischen Informationen, die als Anrufkontextinformationen an die zweite Kommunikationsvorrichtung bereitzustellen sind, von der Rolle des Benutzers abhängt, der der zweiten Kommunikationsvorrichtung zugeordnet ist;
Senden an die zweite Kommunikationsvorrichtung einer Kommunikationsanforderungsnachricht, um eine Sprachkommunikationssitzung als Reaktion auf die Kommunikationsanforderung von der ersten Kommunikationsvorrichtung zu initiieren; und
basierend auf dem Auswählen, Senden an die zweite Kommunikationsvorrichtung zusammen mit der zweiten Kommunikationsanforderung, von Anrufkontextinformationen, die die ausgewählten Elemente der klinischen Informationen einschließen, um der zweiten Kommunikationsvorrichtung zu ermöglichen, die ausgewählten Elemente der klinischen Informationen als eine Ergänzung zu Anruferidentifikationsinformationen für die Sprachkommunikationssitzung darzustellen;
wobei die erste Kommunikationsvorrichtung einen ersten Vorrichtungsprozessor umfasst, der mit prozessorausführbaren Anweisungen konfiguriert ist zum:
Empfangen eines Ereignisalarms;
Anzeigen einer grafische Benutzerschnittstellenanzeige (GUI-Anzeige), die einen Ereignisidentifizierer und Ereigniskontextinformationen einschließt, die in der Ereigniswarnung eingeschlossen sind, zusammen mit Benutzerschnittstellensymbolen zum Annehmen oder Ablehnen der Ereigniswarnung;
Anzeigen einer GUI-Anzeige, um es einer Pflegekraft zu ermöglichen, eine Kommunikationsanforderung bezüglich der Ereigniswarnung zu stellen; und
Übertragen an die Servervorrichtung der Kommunikationsanforderung, als eine Anforderung bezüglich der Ereigniswarnungen, als Reaktion auf das Empfangen einer Benutzereingabe, um die Sprachkommunikationssitzung zu initiieren; und
wobei die zweite Kommunikationsvorrichtung einen zweiten Vorrichtungsprozessor umfasst, der mit prozessorausführbaren Anweisungen konfiguriert ist zum:
Empfangen von der Servervorrichtung der Kommunikationsanforderungsnachricht zusammen mit den Anrufkontextinformationen, die die ausgewählten Elemente der klinischen Informationen einschließen;
Anzeigen einer GUI-Anzeige, die einen Ereignisidentifizierer und die Anrufkontextinformationen zusammen mit Benutzerschnittstellensymbolen zum Annehmen oder Ablehnen der Sprachkommunikationssitzung einschließt; und
Übertragen an die Servervorrichtung einer Nachricht, um die Sprachkommunikationssitzung als Reaktion auf das Empfangen einer Benutzereingabe zu akzeptieren, um Sprachkommunikationssitzung zu akzeptieren.

16. System nach Anspruch 15, wobei der Serverprozessor ferner mit prozessorausführbaren Anweisungen konfiguriert ist zum:
Zuordnen klinisch relevanter Elemente der klinischen Informationen mit einem oder mehreren Ereignisidentifizierern.

17. System nach Anspruch 16, wobei der Serverprozessor ferner mit prozessorausführbaren Anweisungen konfiguriert ist zum:
Formatieren der Elemente klinischer Informationen in ein Format für die Übertragung als Teil der Anrufkontextinformationen an die zweite Kommunikationsvorrichtung.

18. System nach Anspruch 16, wobei der Serverprozessor ferner mit prozessorausführbaren Anweisungen konfiguriert ist zum:
Speichern der Zuordnung der Elemente klinischer Informationen und des einen oder der mehreren Ereignisidentifizierern in einer Datenbank.

19. System nach Anspruch 15, wobei der Serverprozessor ferner mit prozessorausführbaren Anweisungen konfiguriert ist zum:
Bestimmen, ob eine Patientenereigniswarnung empfangen wurde oder basierend auf den empfangenen klinischen Informationen ausgegeben werden sollte; und
Senden an die erste Kommunikationsvorrichtung einer Ereigniswarnung als Reaktion auf das Bestimmen, dass eine Patientenereigniswarnung empfangen wurde oder ausgegeben werden sollte, wobei die Ereigniswarnung den Ereignisidentifizierer und die Ereigniskontextinformationen einschließt.

20. System nach Anspruch 15, wobei das Senden an die zweite Kommunikationsvorrichtung, zusammen mit der Kommunikationsanforderungsnachricht, der Anrufkontextinformationen, die die ausgewählten Elemente der klinischen Informationen einschließen, das Senden an die zweite Kommunikationsvorrichtung, in einem Signal, der Kommunikationsanforderungsnachricht und der Anrufkontextinformationen umfasst.

21. Nicht-flüchtiges prozessorlesbares Speichermedium, das prozessorausführbare Anweisungen darauf gespeichert aufweist, die konfiguriert sind, um einen Prozessor einer Servervorrichtung zu veranlassen, Operationen durchzuführen, umfassend die Schritte eines der Ansprüche 1 bis 5 und 8.

## Revendications

1. Procédé de fourniture d'informations de contexte dans un système de communication médicale, comprenant :
la réception, par un dispositif serveur, d'informations cliniques dans un ou plusieurs fils d'informations provenant d'un ou plusieurs autres dispositifs ;
la détermination, par le dispositif serveur, établissant si une demande de communication provenant d'un premier dispositif de communication pour un second dispositif de communication a été reçue ;
la détermination, par le dispositif serveur, d'un identifiant d'événement associé à la demande de communication provenant du premier dispositif de communication ;
la sélection, par le dispositif serveur, parmi les informations cliniques reçues, d'éléments des informations cliniques associées à l'identifiant d'événement à fournir en tant qu'informations de contexte d'appel au second dispositif de communication, dans lequel le second dispositif de communication est associé à un utilisateur ayant un rôle, et dans lequel la sélection des éléments des informations cliniques à fournir en tant qu'informations de contexte d'appel au second dispositif de communication dépend du rôle de l'utilisateur associé au second dispositif de communication ;
l'envoi, par le dispositif serveur au second dispositif de communication, d'un message de demande de communication pour lancer une session de communication vocale en réponse à la demande de communication provenant du premier dispositif de communication ; et
en fonction de la sélection, l'envoi par le dispositif serveur au second dispositif de communication, conjointement avec le message de demande de communication, d'informations de contexte d'appel comportant les éléments sélectionnés des informations cliniques, pour permettre au second dispositif de communication de présenter les éléments sélectionnés des informations cliniques en complément des informations d'identification d'appelant pour la session de communication vocale.

2. Procédé selon la revendication 1, comprenant en outre :
l'association, par le dispositif serveur, d'éléments cliniquement pertinents des informations cliniques à un ou plusieurs identifiants d'événements.

3. Procédé selon la revendication 2, comprenant en outre :
le formatage, par le dispositif serveur, des éléments d'informations cliniques en un format pour transmission en tant que partie des informations de contexte d'appel au second dispositif de communication.

4. Procédé selon la revendication 2, comprenant en outre :
le stockage, par le dispositif serveur, de l'association des éléments d'informations cliniques et des un ou plusieurs identifiants d'événements dans une base de données.

5. Procédé selon la revendication 1, comprenant en outre :
la détermination établissant si une alerte d'événement patient a été reçue ou doit être émise en fonction des informations cliniques reçues ; et
l'envoi, par le dispositif serveur, au premier dispositif de communication, d'une alerte d'événement en réponse à la détermination qu'une alerte d'événement patient a été reçue ou doit être émise, l'alerte d'événement comportant l'identifiant d'événement et des informations de contexte d'événement.

6. Procédé selon la revendication 5, comprenant en outre :
la réception, par le premier dispositif de communication, de l'alerte d'événement ;
l'affichage, par le premier dispositif de communication, d'un affichage d'interface utilisateur graphique (GUI) identifiant l'identifiant d'événement et les informations de contexte d'événement conjointement avec des icônes d'interface utilisateur pour accepter ou refuser l'alerte d'événement ;
l'affichage, par le premier dispositif de communication, d'un affichage de GUI pour permettre à un soignant de placer une demande de communication relative à l'alerte d'événement ; et
la transmission, par le premier dispositif de communication, au dispositif serveur, de la demande de communication, en tant que demande relative à l'alerte d'événement, en réponse à la réception d'une entrée utilisateur pour lancer la session de communication vocale.

7. Procédé selon la revendication 1, comprenant en outre :
la réception, par le second dispositif de communication, du message de demande de communication conjointement avec les informations de contexte d'appel comportant les éléments sélectionnés des informations cliniques ;
l'affichage, par le second dispositif de communication, d'un affichage d'interface utilisateur graphique (GUI) comportant un identifiant d'événement et les informations de contexte d'appel conjointement avec des icônes d'interface utilisateur pour accepter ou refuser la session de communication vocale ; et
la transmission, par le second dispositif de communication, au dispositif serveur, d'un message pour accepter la session de communication vocale en réponse à la réception d'une entrée utilisateur pour accepter la session de communication vocale.

8. Procédé selon la revendication 1, dans lequel l'envoi, par le dispositif serveur au second dispositif de communication, conjointement avec le message de demande de communication, des informations de contexte d'appel comportant les éléments sélectionnés des informations cliniques comprend l'envoi au second dispositif de communication, dans un signal, du message de demande de communication et des informations de contexte d'appel.

9. Dispositif serveur, comprenant :
un processeur configuré avec des instructions exécutables par processeur pour :
recevoir des informations cliniques dans un ou plusieurs fils d'informations provenant d'un ou plusieurs autres dispositifs ;
déterminer lorsqu'une demande de communication provenant d'un premier dispositif de communication pour un second dispositif de communication a été reçue ;
déterminer un identifiant d'événement associé à la demande de communication provenant du premier dispositif de communication ;
sélectionner, parmi les informations cliniques reçues, des éléments des informations cliniques associées à l'identifiant d'événement à fournir en tant qu'informations de contexte d'appel au second dispositif de communication, dans lequel le second dispositif de communication est associé à un utilisateur ayant un rôle, et dans lequel la sélection des éléments des informations cliniques à fournir en tant qu'informations de contexte d'appel au second dispositif de communication dépend du rôle de l'utilisateur associé au second dispositif de communication ;
envoyer un message de demande de communication au second dispositif de communication, un message de demande de communication pour lancer une session de communication vocale en réponse à la demande de communication provenant du premier dispositif de communication ; et
en fonction de la sélection, envoyer au second dispositif de communication, conjointement avec la demande de communication, des informations de contexte d'appel comportant les éléments sélectionnés des informations cliniques, pour permettre au second dispositif de communication de présenter les éléments sélectionnés des informations cliniques en complément des informations d'identification d'appelant pour la session de communication vocale.

10. Dispositif serveur selon la revendication 9, dans lequel le processeur est en outre configuré avec des instructions exécutables par processeur pour :
associer des éléments cliniquement pertinents des informations cliniques à un ou plusieurs identifiants d'événement.

11. Dispositif serveur selon la revendication 10, dans lequel le processeur est en outre configuré avec des instructions exécutables par processeur pour :
formater les éléments d'informations cliniques en un format pour transmission en tant que partie des informations de contexte d'appel au second dispositif de communication.

12. Dispositif serveur selon la revendication 10, dans lequel le processeur est en outre configuré avec des instructions exécutables par processeur pour :
stocker l'association des éléments d'informations cliniques et des un ou plusieurs identifiants d'événements dans une base de données.

13. Dispositif serveur selon la revendication 12, dans lequel le processeur est en outre configuré avec des instructions exécutables par processeur pour :
déterminer si une alerte d'événement patient a été reçue ou doit être émise en fonction des informations cliniques reçues ; et
envoyer, au premier dispositif de communication, une alerte d'événement en réponse à la détermination qu'une alerte d'événement patient a été reçue ou doit être émise, l'alerte d'événement comportant l'identifiant d'événement et des informations de contexte d'événement.

14. Dispositif serveur selon la revendication 9, dans lequel l'envoi au second dispositif de communication, conjointement avec le message de demande de communication, des informations de contexte d'appel comportant les éléments sélectionnés des informations cliniques comprend l'envoi au second dispositif de communication, dans un signal, du message de demande de communication et des informations de contexte d'appel.

15. Système, comprenant :
un dispositif serveur ;
un premier dispositif de communication ; et
un second dispositif de communication,
dans lequel le dispositif serveur comprend un processeur de serveur configuré avec des instructions exécutables par processeur pour :
recevoir des informations cliniques dans un ou plusieurs fils d'informations provenant d'un ou plusieurs autres dispositifs ;
déterminer lorsqu'une demande de communication provenant du premier dispositif de communication pour le second dispositif de communication a été reçue ;
déterminer un identifiant d'événement associé à la demande de communication provenant du premier dispositif de communication ;
sélectionner, parmi les informations cliniques reçues, des éléments des informations cliniques associées à l'identifiant d'événement à fournir en tant qu'informations de contexte d'appel au second dispositif de communication, dans lequel le second dispositif de communication est associé à un utilisateur ayant un rôle, et dans lequel la sélection des éléments des informations cliniques à fournir en tant qu'informations de contexte d'appel au second dispositif de communication dépend du rôle de l'utilisateur associé au second dispositif de communication ;
envoyer, au second dispositif de communication, un message de demande de communication pour lancer une session de communication vocale en réponse à la demande de communication provenant du premier dispositif de communication ; et
en fonction de la sélection, envoyer au second dispositif de communication, conjointement avec la seconde demande de communication, des informations de contexte d'appel comportant les éléments sélectionnés des informations cliniques, pour permettre au second dispositif de communication de présenter les éléments sélectionnés des informations cliniques en complément des informations d'identification d'appelant pour la session de communication vocale ;
dans lequel le premier dispositif de communication comprend un processeur de premier dispositif configuré avec des instructions exécutables par processeur pour :
recevoir une alerte d'événement ;
afficher un affichage d'interface utilisateur graphique (GUI) comportant un identifiant d'événement et des informations de contexte d'événement compris dans l'alerte d'événement conjointement avec des icônes d'interface utilisateur pour accepter ou refuser l'alerte d'événement ;
afficher un affichage de GUI pour permettre à un soignant de placer une demande de communication relative à l'alerte d'événement ; et
transmettre au dispositif serveur la demande de communication, en tant que demande relative aux alertes d'événement en réponse à la réception d'une entrée utilisateur pour lancer la session de communication vocale ; et
dans lequel le second dispositif de communication comprend un processeur de second dispositif configuré avec des instructions exécutables par processeur pour :
recevoir, en provenance du dispositif serveur, le message de demande de communication conjointement avec les informations de contexte d'appel comportant les éléments sélectionnés des informations cliniques ;
afficher un affichage de GUI comportant un identifiant d'événement et les informations de contexte d'appel conjointement avec des icônes d'interface utilisateur pour accepter ou refuser la session de communication vocale ; et
transmettre au dispositif serveur un message pour accepter la session de communication vocale en réponse à la réception d'une entrée utilisateur pour accepter la session de communication vocale.

16. Système selon la revendication 15, dans lequel le processeur de serveur est en outre configuré avec des instructions exécutables par processeur pour :
associer des éléments cliniquement pertinents des informations cliniques à un ou plusieurs identifiants d'événement.

17. Système selon la revendication 16, dans lequel le processeur de serveur est en outre configuré avec des instructions exécutables par processeur pour :
formater les éléments d'informations cliniques en un format pour transmission en tant que partie des informations de contexte d'appel au second dispositif de communication.

18. Système selon la revendication 16, dans lequel le processeur de serveur est en outre configuré avec des instructions exécutables par processeur pour :
stocker l'association des éléments d'informations cliniques et des un ou plusieurs identifiants d'événements dans une base de données.

19. Système selon la revendication 15, dans lequel le processeur de serveur est en outre configuré avec des instructions exécutables par processeur pour :
déterminer si une alerte d'événement patient a été reçue ou doit être émise en fonction des informations cliniques reçues ; et
envoyer, au premier dispositif de communication, une alerte d'événement en réponse à la détermination qu'une alerte d'événement patient a été reçue ou doit être émise, l'alerte d'événement comportant l'identifiant d'événement et les informations de contexte d'événement.

20. Système selon la revendication 15, dans lequel l'envoi au second dispositif de communication, conjointement avec le message de demande de communication, des informations de contexte d'appel comportant les éléments sélectionnés des informations cliniques comprend l'envoi au second dispositif de communication, dans un signal, du message de demande de communication et des informations de contexte d'appel.

21. Support de stockage non transitoire lisible par processeur sur lequel sont stockées des instructions exécutables par processeur configurées pour amener un processeur d'un dispositif serveur à réaliser des opérations comprenant les étapes selon l'une quelconque des revendications 1 à 5 et 8.
